# EUROPEAN PATENT APPLICATION

(11) **EP 2 198 878 A1**
(43) Date of publication of application: **23.06.2010**
(21) Application number: 09013790.2
(22) Date of filing: 03.11.2009
(51) Int. Cl.: A61K 38/08, A61K 38/10, A61P 35/00

(54) **Polypeptide bombesin antagonists**

(30) Priority: 18.12.2008 US 316995
(71) Applicant: University Of Miami, Miami, Florida 33136 (US); The United States of America Represented by the Department of Veterans Affairs, Washington DC 20430 (US)
(72) Inventor: Shally, Andrew V.,, Miami Beach, FL 33140 (US); Szepeshazi, Karoly, Aventura FL 33180 (US); Cai, Ren-Zhi, Metairie, LA 70003 (US)
(74) Representative: Luderschmidt, Schüler & Partner

(57) **Abstract**

There is provided a method of treating human hepatomas utilizing bombesin antagonist pseudopeptides of Formula I:

X-A¹-A²-Trp-Ala-Val-Gly-His-Leu-Ψ-A⁹ -Q (SEQ ID NO: 32)

wherein X is hydrogen, Hca, Hna, Hpp, Mpp or Naa,
A¹ is a D-, L- or DL-amino acid residue selected from the group consisting of Phe, p-HI-Phe, pGlu, Nal, Pal, Tpi, unsubstituted Trp or Trp substituted in the benzene ring by one or more members selected from the group consisting of F, Cl, Br, NH₂ or C₁₋₃ alkyl; or a peptide bond linking the acyl moiety of X (where present) to the alpha amino moiety of A²,
A² is Gin, Glu [-], Glu (Y), or His, wherein
[-] is a single bond, linking the gamma carboxyl moiety or the 3-propionyl moiety of A² with the alpha amino moiety of A¹,
Y is
a) -OR⁵ wherein R⁵ is hydrogen, C₃ alkyl or phenyl; or
b) R₆ 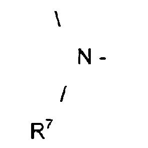 wherein R⁶ is hydrogen or C₁₋₃ alkyl; R⁷ is hydrogen, C₁₋₃ alkyl or-NHCONH₂. Leu-Ψ- is a reduced form of Leu wherein the C = O moiety of Leu is instead -CH₂- such that the bond of this -CH₂- moiety with the alpha amino moiety of the adjacent A⁹ residue is a pseudopeptide bond. Suitably A⁹ is Tac, MTac, or DMTac,
Q is NH₂ or -OQ' where Q' is hydrogen, C₁₋₁₀ alkyl, phenyl or phenyl-C₁₋₁₀ -alkyl; and the pharmaceutically acceptable acids or salts thereof.

SEQUENCE LISTING

<110> SCHALLY, ANDREW V.
      SZEPESHAZI, KAROLY

<120> POLYPEPTIDE BOMBESIN ANTAGONISTS

<130> SHAL3.0-034

<140>
<141>

<160> 33

<170> PatentIn version 3.5

<210> 1
<211> 14
<212> PRT
<213> Bombina bombina


<220>
<221> MOD_RES
<222> (1)..(1)
<223> pGlu

<400> 1
Glu Gln Arg Leu Gly Asn Gln Trp Ala Val Gly His Leu Met
1               5                   10


<210> 2
<211> 10
<212> PRT
<213> Homo sapiens

<400> 2
Gly Asn His Trp Ala Val Gly His Leu Met
1               5                   10


<210> 3
<211> 9
<212> PRT
<213> Sus sp.

<400> 3
Asn His Trp Ala Val Gly His Leu Met
1               5


<210> 4
<211> 9
<212> PRT
<213> Bombina bombina


<220>
<221> MOD_RES
<222> (1)..(1)
<223> pGlu

<400> 4
Glu Gln Trp Ala Val Gly His Leu Met
1               5

<210> 5
<211> 11
<212> PRT
<213> Bos sp.

<400> 5
Arg Pro Lys Pro Gln Gln Phe Phe Gly Leu Leu
1               5                   10


<210> 6
<211> 14
<212> PRT
<213> Artificial Sequence

<220>
<223> Description of Artificial Sequence: Synthetic
      peptide


<220>
<221> MOD_RES
<222> (1)..(1)
<223> pGlu

<220>
<221> MOD_RES
<222> (13)..(13)
<223> Pseudo-Leu

<400> 6
Glu Gln Arg Leu Gly Asn Gln Trp Ala Val Gly His Leu Leu
1               5                   10


<210> 7
<211> 9
<212> PRT
<213> Artificial Sequence

<220>
<223> Description of Artificial Sequence: Synthetic
      peptide


<220>
<221> MOD_RES
<222> (1)..(1)
<223> Hca

<220>
<221> MOD_RES
<222> (8)..(8)
<223> Pseudo-Leu

<220>
<221> MOD_RES
<222> (9)..(9)
<223> Tac

<400> 7
Xaa Gln Trp Ala Val Gly His Leu Xaa
1               5


<210> 8
<211> 9
<212> PRT

<213> Artificial Sequence

<220>
<223> Description of Artificial Sequence: Synthetic
      peptide

<220>
<221> MOD_RES
<222> (1)..(1)
<223> Tpi

<220>
<221> MOD_RES
<222> (8)..(8)
<223> Pseudo-Leu

<220>
<221> MOD_RES
<222> (9)..(9)
<223> Tac

<400> 8
Xaa Gln Trp Ala Val Gly His Leu Xaa
1               5

<210> 9
<211> 9
<212> PRT
<213> Artificial Sequence

<220>
<223> Description of Artificial Sequence: Synthetic
      peptide

<220>
<221> MOD_RES
<222> (1)..(1)
<223> Tpi

<220>
<221> MOD_RES
<222> (8)..(8)
<223> Pseudo-Leu

<220>
<221> MOD_RES
<222> (9)..(9)
<223> DMTac

<400> 9
Xaa Gln Trp Ala Val Gly His Leu Xaa
1               5

<210> 10
<211> 9
<212> PRT
<213> Artificial Sequence

<220>
<223> Description of Artificial Sequence: Synthetic
      peptide

<220>

<221> MOD_RES
<222> (1)..(1)
<223> Pal

<220>
<221> MOD_RES
<222> (8)..(8)
<223> Pseudo-Leu

<400> 10
Xaa Gln Trp Ala Val Gly His Leu Cys
1               5


<210> 11
<211> 9
<212> PRT
<213> Artificial Sequence

<220>
<223> Description of Artificial Sequence: Synthetic
      peptide


<220>
<221> MOD_RES
<222> (1)..(1)
<223> Tpi

<220>
<221> MOD_RES
<222> (8)..(8)
<223> Pseudo-Leu

<400> 11
Xaa Gln Trp Ala Val Gly His Leu Cys
1               5


<210> 12
<211> 9
<212> PRT
<213> Artificial Sequence

<220>
<223> Description of Artificial Sequence: Synthetic
      peptide


<220>
<221> MOD_RES
<222> (1)..(1)
<223> Hca

<220>
<221> MOD_RES
<222> (8)..(8)
<223> Pseudo-Leu

<400> 12
Xaa Gln Trp Ala Val Gly His Leu Cys
1               5


<210> 13
<211> 6
<212> PRT
<213> Artificial Sequence

<220>
<223> Description of Artificial Sequence: Synthetic
      peptide

<400> 13
Trp Ala Val Gly His Leu
1               5


<210> 14
<211> 8
<212> PRT
<213> Artificial Sequence

<220>
<223> Description of Artificial Sequence: Synthetic
      peptide


<220>
<221> MOD_RES
<222> (1)..(1)
<223> Pal

<220>
<221> MOD_RES
<222> (8)..(8)
<223> Pseudo-Leu

<400> 14
Xaa Gln Trp Ala Val Gly His Leu
1               5


<210> 15
<211> 9
<212> PRT
<213> Artificial Sequence

<220>
<223> Description of Artificial Sequence: Synthetic
      peptide


<220>
<221> MOD_RES
<222> (1)..(1)
<223> Pal

<220>
<221> MOD_RES
<222> (8)..(8)
<223> Pseudo-Leu

<220>
<221> MOD_RES
<222> (9)..(9)
<223> Tac

<400> 15
Xaa Gln Trp Ala Val Gly His Leu Xaa
1               5


<210> 16
<211> 7
<212> PRT

<213> Artificial Sequence

<220>
<223> Description of Artificial Sequence: Synthetic
      peptide


<220>
<221> MOD_RES
<222> (5)..(5)
<223> His(Bom)

<220>
<221> MOD_RES
<222> (6)..(6)
<223> Pseudo-Leu

<220>
<221> MOD_RES
<222> (7)..(7)
<223> Cys(But)

<400> 16
Trp Ala Val Gly His Leu Cys
1               5


<210> 17
<211> 9
<212> PRT
<213> Artificial Sequence

<220>
<223> Description of Artificial Sequence: Synthetic
      peptide


<220>
<221> MOD_RES
<222> (1)..(1)
<223> Pal

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Cys(But)

<400> 17
Xaa Gln Trp Ala Val Gly His Leu Cys
1               5


<210> 18
<211> 9
<212> PRT
<213> Artificial Sequence

<220>

<223> Description of Artificial Sequence: Synthetic
      peptide

<220>
<221> MOD_RES
<222> (1)..(1)
<223> Tpi

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Cys(But)

<400> 18
Xaa Gln Trp Ala Val Gly His Leu Cys
1               5

<210> 19
<211> 9
<212> PRT
<213> Artificial Sequence

<220>
<223> Description of Artificial Sequence: Synthetic
      peptide

<220>
<221> MOD_RES
<222> (1)..(1)
<223> Hca

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Cys(But)

<400> 19
Xaa Gln Trp Ala Val Gly His Leu Cys
1               5

<210> 20
<211> 9
<212> PRT
<213> Artificial Sequence

<220>
<223> Description of Artificial Sequence: Synthetic
      peptide

<220>
<221> MOD_RES
<222> (1)..(1)
<223> Pal

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Tac

<400> 20
Xaa Gln Trp Ala Val Gly His Leu Xaa
1               5

<210> 21
<211> 9
<212> PRT
<213> Artificial Sequence

<220>
<223> Description of Artificial Sequence: Synthetic
      peptide

<220>
<221> MOD_RES
<222> (1)..(1)
<223> Tpi

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Tac

<400> 21
Xaa Gln Trp Ala Val Gly His Leu Xaa
1               5

<210> 22
<211> 9

<212> PRT
<213> Artificial Sequence

<220>
<223> Description of Artificial Sequence: Synthetic
      peptide


<220>
<221> MOD_RES
<222> (1)..(1)
<223> Hca

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Tac

<400> 22
Xaa Gln Trp Ala Val Gly His Leu Xaa
1               5


<210> 23
<211> 8
<212> PRT
<213> Artificial Sequence

<220>
<223> Description of Artificial Sequence: Synthetic
      peptide


<220>
<221> MOD_RES
<222> (6)..(6)
<223> His(Bom)

<220>
<221> MOD_RES
<222> (7)..(7)
<223> Pseudo-Leu

<220>
<221> MOD_RES
<222> (8)..(8)
<223> Cys(But)

<400> 23
Gln Trp Ala Val Gly His Leu Cys
1               5


<210> 24
<211> 7
<212> PRT
<213> Artificial Sequence

<220>
<223> Description of Artificial Sequence: Synthetic
      peptide

<220>
<221> MOD_RES
<222> (5)..(5)
<223> His(Bom)

<220>
<221> MOD_RES
<222> (6)..(6)
<223> Pseudo-Leu

<220>
<221> MOD_RES
<222> (7)..(7)
<223> Pen(But)

<400> 24
Trp Ala Val Gly His Leu Xaa
1               5

<210> 25
<211> 9
<212> PRT
<213> Artificial Sequence

<220>
<223> Description of Artificial Sequence: Synthetic
      peptide

<220>
<221> MOD_RES
<222> (1)..(1)
<223> Tpi

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Pen(But)

<400> 25
Xaa Gln Trp Ala Val Gly His Leu Xaa
1               5

<210> 26
<211> 9
<212> PRT
<213> Artificial Sequence

<220>
<223> Description of Artificial Sequence: Synthetic
      peptide

<220>
<221> MOD_RES
<222> (1)..(1)
<223> Pal

<220>
<221> MOD_RES
<222> (7)..(7)
<223> His(Bom)

<220>
<221> MOD_RES
<222> (8)..(8)
<223> Pseudo-Leu

<400> 26
Xaa Gln Trp Ala Val Gly His Leu Cys
1               5


<210> 27
<211> 9
<212> PRT
<213> Artificial Sequence

<220>
<223> Description of Artificial Sequence: Synthetic
      peptide


<220>
<221> MOD_RES
<222> (1)..(1)
<223> Pal

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Cys(Bz)

<400> 27
Xaa Gln Trp Ala Val Gly His Leu Cys
1               5


<210> 28
<211> 9
<212> PRT
<213> Artificial Sequence

<220>
<223> Description of Artificial Sequence: Synthetic
      peptide


<220>

<221> MOD_RES
<222> (1)..(1)
<223> Tpi

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Cys(Bz)

<400> 28
Xaa Gln Trp Ala Val Gly His Leu Cys
1               5


<210> 29
<211> 9
<212> PRT
<213> Artificial Sequence

<220>
<223> Description of Artificial Sequence: Synthetic
      peptide


<220>
<221> MOD_RES
<222> (1)..(1)
<223> Hca

<220>
<221> MOD_RES
<222> (7)..(7)
<223> His(Bom)

<220>
<221> MOD_RES
<222> (8)..(8)
<223> Pseudo-Leu

<220>
<221> MOD_RES
<222> (9)..(9)
<223> Cys(Bz)

<400> 29
Xaa Gln Trp Ala Val Gly His Leu Cys
1               5


<210> 30
<211> 10
<212> PRT
<213> Artificial Sequence

<220>
<223> Description of Artificial Sequence: Synthetic
      peptide

<220>
<221> MOD_RES
<222> (1)..(1)
<223> Any amino acid analog or not present

<220>
<221> MOD_RES
<222> (2)..(2)
<223> Any D-, L- or DL- amino acid residue selected from
      Phe, p-HI-Phe, pGlu, Nal, Pal, Tpi, Trp or not present

<220>
<221> MOD_RES
<222> (3)..(3)
<223> Gln, Glu or His

<220>
<221> MOD_RES
<222> (9)..(9)
<223> Pseudo-Leu

<220>
<221> MOD_RES
<222> (10)..(10)
<223> Tac, MTac or DMTac

<220>
<223> see specification as filed for detailed description of
      substitutions and preferred embodiments

<400> 30
Xaa Xaa Xaa Trp Ala Val Gly His Leu Xaa
1               5                   10


<210> 31
<211> 9
<212> PRT
<213> Artificial Sequence

<220>
<223> Description of Artificial Sequence: Synthetic
      peptide


<220>
<221> MOD_RES
<222> (1)..(1)
<223> Any D-, L- or DL- amino acid residue selected from
      Phe, p-HI-Phe, pGlu, Nal, Pal, Tpi, Trp or not present

<220>
<221> MOD_RES
<222> (2)..(2)
<223> Gln, Glu or His

<220>
<221> MOD_RES
<222> (8)..(8)
<223> Pseudo-Leu

<220>
<221> MOD_RES
<222> (9)..(9)
<223> Tac, MTac or DMTac

<220>

<223> see specification as filed for detailed description of
      substitutions and preferred embodiments

<400> 31
Xaa Xaa Trp Ala Val Gly His Leu Xaa
1               5


<210> 32
<211> 10
<212> PRT
<213> Artificial Sequence

<220>
<223> Description of Artificial Sequence: Synthetic
      peptide


<220>
<221> MOD_RES
<222> (1)..(1)
<223> Hca, Hna, Hpp, Mpp, Naa or not present

<220>
<221> MOD_RES
<222> (2)..(2)
<223> Any D-, L- or DL- amino acid residue selected from
      Phe, p-HI-Phe, pGlu, Nal, Pal, Tpi, Trp or not present

<220>
<221> MOD_RES
<222> (3)..(3)
<223> Gln, Glu or His

<220>
<221> MOD_RES
<222> (9)..(9)
<223> Pseudo-Leu

<220>
<221> MOD_RES
<222> (10)..(10)
<223> Tac, MTac or DMTac

<220>
<223> see specification as filed for detailed description of
      substitutions and preferred embodiments

<400> 32
Xaa Xaa Xaa Trp Ala Val Gly His Leu Xaa
1               5                   10


<210> 33
<211> 10
<212> PRT
<213> Artificial Sequence

<220>
<223> Description of Artificial Sequence: Synthetic
      peptide

<220>
<221> MOD_RES
<222> (1)..(1)
<223> Hca, Hna, Hpp, Mpp, Naa or not present

<220>

<221> MOD_RES
<222> (2)..(2)
<223> Any D-, L- or DL- amino acid residue selected from
      Phe, p-HI-Phe, pGlu, Nal, Pal, Tpi, Trp or not present

<220>
<221> MOD_RES
<222> (3)..(3)
<223> Gln, Glu or His

<220>
<221> MOD_RES
<222> (9)..(9)
<223> Pseudo-Leu

<220>
<221> MOD_RES
<222> (10)..(10)
<223> Cys or Pen

<220>
<223> see specification as filed for detailed description of
      substitutions and preferred embodiments

<400> 33
Xaa Xaa Xaa Trp Ala Val Gly His Leu Xaa
1               5                   10

## Description

This invention was made with Government support, awarded by the Veterans Affairs Department. The U.S. Government has certain rights in this application.

### FIELD OF THE INVENTION

The present invention is directed to novel peptides which influence the growth of hepatocellular cancer tumors in humans. More specifically, the present invention relates to bombesin antagonists which are (ψ) pseudopeptides containing a Tac, MTac, or DMTac residue at the C terminal position, the salts thereof, and pharmaceutical compositions, methods of synthesizing these peptides and methods of use pertaining to these peptides. These peptides possess antagonist properties against bombesin or bombesin-like peptides.

### BACKGROUND OF THE INVENTION

This invention relates to polypeptide compounds which possess antagonist properties against bombesin or bombesin-like peptides (such as gastrin releasing peptide (GRP), Neuromedin C and the like) hereinafter referred to as bombesin antagonist properties and which are of value, for example in the treatment of malignant diseases in warm-blooded organisms such as man. The invention includes novel polypeptide compounds and processes for their manufacture; novel pharmaceutical compositions containing said polypeptide compounds and processes for the manufacture of medicaments containing them for use in producing a bombesin antagonist effect in warm-blooded organisms such as man.

Bombesin is a tetradecapeptide amide which was first isolated from the skin of the frog Bombina bombina (Anastasi, Erspamer and Bucci, Experientia. 1971 , 22, 166). It is known that bombesin is a potent mitogen for mouse Swiss 3T3 fibroblast cells (Rozengurt and Sinnett-Smith, Proc. Natl. Acad. Sci. USA. 1983, 80, 2936) and that it stimulates amylase secretion from guinea pig pancreatic acini (Jensen, Jones, Folkers and Gardner, Nature. 1984, 309, 61 ). It is also known that bombesin-like peptides are produced and secreted by human small-cell lung cancer (SCLC) cells (Moody, Pert, Gazdar, Carney and Minna, Science. 1981 , 214. 1246), that exogenously added bombesin-like peptides can stimulate the growth of human SCLC cells jn vitro (Carney, Cuttita, Moody and Minna. Cancer Research. 1987, 47, 821 ) and that a monoclonal antibody specific for the C-Terminus region of bombesin and GRP can block binding of GRP to its receptors and prevent the growth of human SCLC cells both jn vitro and jn vivo (Cuttita, Carney, Mulshine, Moody, Fedorko, Fischler and Minna, Nature. 1985, 316. 823).

GRP which has bombesin-like properties is a widely distributed peptide amide containing 27 amino acids isolated from the porcine gut (McDonald, Jornvall, Nilsson, Vagne, Ghatei, Bloom and Mutt, Biochem. Biophvs. Res. Commun.. 1979, 90, 227) in which the C-terminus amino acid sequence is almost identical to that of bombesin. Neuromedin C is a decapeptide amide, the structure of which is identical to the last ten amino acids in the C-terminus region of GRP, which has been isolated from the canine small intestine (Reeve, Walsh, Chew, Clark, Hawke and Shively, J. Biol. Chem.. 1983, 258. 5582). GRP simulates a variety of biological responses, including the release of gastrin in the i stemic circulation. It also functions as a growth factor in 3T3 mouse fibroblasts and small cell lung cancer (SCLC) cell. So GRP has been proposed to play a direct pathophysiological role in the development of SCLC via an autocrine growth mechanism.

The structures of bombesin, Neuromedin C and Carboxyl-terminal nonapeptide of GRP are shown below:
Bombesin: pGlu-Gln-Arg-Leu-Gly-Asn-Gln-Trp-Ala-Val-Gly-His-Leu-Met-NH₂ [SEQ ID NO: 1]
Neuromedin C: H-Gly-Asn-His-Trp-Ala-Val-Gly-His-Leu-Met-NH₂ [SEQ ID NO: 2]
C-terminal nonapeptide of GRP: Asn-His-Trp-Ala-Val-Gly-His-Leu-Met-NH₂ [SEQ ID NO: 3]

The search for other amphibian bombesin-like peptides led to the isolation of Litorin, a nonapeptide (pGlu-Gln-Trp-Ala-Val-Gly-His-Leu-Met-NH₂ [SEQ ID NO: 4] ) in the skin of frog from Papua, New Guinea which proves to be an extremely potent bombesin analogues (Yasukara et al., Chem. Pharm. Bull., 1979, 27, 492). The studies on bombesin analogues revealed that a minimum segment of the 9 amino acid residues from position 6 to 14 of bombesin possessed the full spectrum of bombesin activity.

Several kinds of bombesin antagonists have now been characterized. Substance P (Arg-Pro-Lys-Pro-Gln-Gln-Phe-Phe-Gly-Leu-Met-NH₂ [SEQ ID NO: 5] )which has slight amino acid sequence homology with bombesin does not inhibit the binding of bombesin and bombesin-like peptides, but Substance P analogues modified by the replacement of several of L-amino acids with D-amino acids such as (D-Arg¹, D-Pro², D-^{Trp7,8}, Leu¹¹) Substance P and (D-Arg¹, D-Phe⁵, D-Trp^{7,9}, Leu¹¹) Substance P, (Moody et al., Fed. Proceedings, 1987, 46» 2201 ) were found to block the secreting of bombesin in pancreatic acinar cells and to antagonize the growth- promoting effects of bombesin in Swiss 3T3 cells. Two types of bombesin antagonists derived from bombesin, for instance, (D-Phe⁶, D-Phe¹²) bombesin, and [Leu¹³-ψ-Leu¹⁴] bombesin [SEQ ID NO: 6] (Coy et al., J. Biol. Chem.. 1988, 263. 5056 and peptides, 1989, 1Q, 587) have proved to be potent in vitro and in vivo inhibitors of bombesin response.
Another type of bombesin antagonist revealed by Heimbrook et al., (Bio. Chem., 1989, 264. 1 1258) is N-acetyl-GRP(20-26) and its analogues, wherein the C-terminal methionine residue is deleted from GRP(20-27) analogues. Coy [J. Biol. Chem. 264. 1989, 25., 14691 ] reported that some short chain bombesin antagonists based on Litorin sequence such as [D-Phe⁶, Leu¹³- ψ-Phe¹⁴] bombesin- (6-14) and [D-Phe⁶, Leu¹³-ψ-Leu¹⁴] bombesin-(6-14) exhibited much more potency than their corresponding parent peptide [Leu¹³-ψ-Leu¹⁴] bombesin [SEQ ID NO: 6].

Linear (non-cyclic) bombesin analogues of GRP and amphibian bombesin optionally having a CH₂-NH non-peptide bond are described in PCT Patent Application WO 90/03980 (and related analogues in WO 91/02746). These analogues, said to act as inhibitors of natural bombesin peptides, have the formula

R₁-A⁰-A¹-A²-Trp-A⁴-A⁵-A⁶-A⁷-A⁸-W

where R₁ and R₂ are H, A⁰ may be deleted; among the many possible amino acids at each position, A¹ may = D-Phe, D-Trp, or D-Nal; A² may = Gin; A⁴ may = Ala; A⁵ may = Val; A⁶ may = Gly; A⁷ may = His; and W = 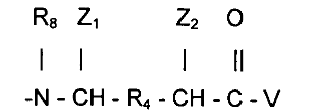 where R₄ = CH₂-NH; in some circumstances, Z. may = the identifying side chain of Leu, i.e., -CH₂CH(CH₃)₂; Z₂ may = the identifying side chain of Cys or Met, i.e., -CH₂-SH or (CH₂)₂-S-CH₃; V = N(R₆)R₇, where R₆, R₇, and R₈ may = H; R, and R₂ may = H or COE₁, where E₁ may = C₁₋₂₀ alkyl.
Linear peptide analogs of bombesin are also described in EP 0 309 297. These peptides may have C-terminal Met residue and a [CH₂-NH] pseudopeptide bond between the C-terminal and its adjacent residue.
Further work in this field was disclosed in US Patent 5365094, the disclosure of which is incorporated herein by reference and substantially reproduced in the present application. That patent discloses that the novel compounds therein are useful in the treatment of certain mammalian cancers, in particular lung cancer, colon cancer and gastric cancer. There is no mention of their utility in other cancers. To date there has been no disclosure of an effective suppressant of hepatocellular cancer tumors.
Bogden et al US 5736517 deals with bombesin antagonists in the treatment of colon, breast or prostate cancer, but makes no mention of liver cancer.Bogden et al. US 6083915, is directed to both hepta and nonapeptide bombesin antagonists and their use in treatment of mammalian hepatomas (liver cancer). This patent discloses the use of peptides which differ from the substituents at the N- (left) and C- (right) termini from those claimed herein and disclosed in US Patent 5365094.
Krstenansky US 5834 433 is directed to the treatment of SCLC and prostatic cancers not liver cancers. The substituents at the N terminus of the present application, might be considered to fall under a general designation of alkanoyl. However the term "alkanoyl" seems to be used in its strictest form, not allowing for aromatic substitution of an alkanoyl chain. Furthermore there is no hint of a pseudo peptide linkage to the substituent at the other end of the chain and certainly not a heterocyclic one. Burman US 6989371 is directed to novel anti cancer compounds and mentions certain compounds disclosed herein, but does not mention liver cancer. Bedian US 7338660 mentions certain compounds disclosed herein and liver cancer, but not juxtaposed in a manner by might suggest the present invention to one skilled in the art.

### SUMMARY OF THE INVENTION

The present invention discloses certain polypeptides which are potent bombesin antagonists and may be utilized in the inhibition of the growth of hepatomas; methods of synthesizing these polypeptides; and the utilization of these compounds in the formation of pharmaceutical compositions comprising said polypeptides and of said polypeptides and compositions as pharmaceutically active agents and the use thereof in combating hepatomas.

### A. Synthetic Peptides

More particularly, a first embodiment provides potent bombesin antagonist pseudopeptides of Formula I:

X-A¹-A²-Trp-Ala-Val-Gly-His-Leu-ψ-A⁹ -Q (SEQ ID NO: 30)

wherein X is hydrogen, Ac or an amino acid analog such as Hca, Hna, Hpp, Mpp or Naa,
A¹ is a D-, L- or DL-amino acid residue selected from the group consisting of Phe, p-HI-Phe, pGlu, Nal, Pal, Tpi, unsubstituted Trp or Trp substituted in the benzene ring by one or more members selected from the group consisting of F, Cl, Br, NH₂ or C₁₋₃ alkyl; or a peptide bond linking the acyl moiety of X (where present) to the alpha amino moiety of A²;
A² is Gin, Glu [-], Glu (Y), or His, wherein
[-] is a single bond, linking the gamma carboxyl moiety or the 3-propionyl moiety of A² with the alpha amino moiety of A¹,
Y is
a) -OR⁵ wherein R⁵ is hydrogen, C ₃ alkyl or phenyl; or
b) 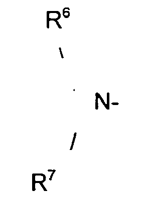 wherein R⁶ is hydrogen or C₁₋₃ alkyl; R⁷ is hydrogen, C₁₋₃ alkyl or -NHCONH₂. Leu-ψ- is a reduced form of Leu wherein the C = O moiety of Leu is instead -CH₂-such that the bond of this -CH₂- moiety with the alpha amino moiety of the adjacent A⁹ residue is a pseudopeptide bond. Suitably A⁹ is Tac, MTac, or DMTac; Q is NH₂ or -OQ¹ where Q¹ is hydrogen, C₁₋₁₀ alkyl, phenyl or phenyl-C₁₋₁₀ -alkyl; and the pharmaceutically acceptable acids or salts thereof.

Where A⁹ = Tac, MTac or DMTac, there is present at A⁹ a 5-membered heterocyclic ring. This ring is generally formed by oxidizing the A⁹ residue's side chain at some point in the synthesis of the Formula I nonapeptide, preferably with formaldehyde or acetaldehyde to cyclize the sidechain with the alpha amino group of A⁹. The identity of the resulting cyclized A⁹ residue depends on the identity of the original, unoxidized A⁹ and the oxidizing reagent used thereon.Thus, when the -CH₂-SH group of Cys⁹ cyclizes with the Cys⁹ alpha amino group adjacent the Leu-ψ-pseudopeptide linkage in a reaction with formaldehyde, the resulting ring has the structure of Formula IIA (shown here as the -Leu⁸-ψ-Tac⁹-NH₂ fragment of the Formula I nonapeptide):

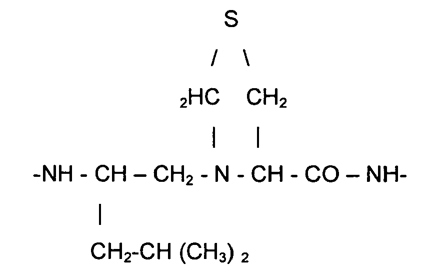

In a preferred form of these peptides, X is Hca, Hna, Paa, Mpp, Hpp or Naa; A¹ is a peptide bond linking the acyl moiety of X to the alpha amino moiety of A² A² is Gln and A⁹ is Tac and Q is NH₂.

### B. Synthetic Methods

The bombesin antagonists of Formula I may be synthesized by solid phase synthesis. In a first protocol, all the amino acids are sequentially linked to one another after the C-terminal residue has been linked to the resin support phase. Once all the amino acyl residues are linked to the resin, the pseudopeptide's 5-membered heterocyclic ring is formed by reaction of the side chain of the C- terminal residue with an oxidizing reagent. The pseudopeptide is then subjected to HF treatment to remove it from the resin support phase. This reaction also removes the side chain protecting groups.

In a second protocol, the pseudopeptides of Formula I may be synthesized as two fragments which are built by solid phase or liquid phase. A tripeptide containing C-terminal is linked with an oligopeptide to form the entire Formula I bombesin antagonist.

The 5 member heterocyclic ring is formed by reaction of the A⁹ side chain with an oxidizing reagent. The peptide is then subjected to HF treatment, which removes all the amino acyl residue side chain protecting groups and cleaves the peptide from the resin.

### C. Medical Applications

The pseudopeptides of Formula I may be employed in pharmaceutical compositions to treat hepatocellular mammalian cancers as well as other conditions, by administering an effective dose of the pseudopeptide, or a therapeutically acceptable acid or salt thereof in a pharmaceutical carrier. They may be administered with a pharmaceutically acceptable carrier at dosages of from about 1 to 1,000 micrograms per kg of body weight daily. Such a composition may be administered parenterally, intravenously, subcutaneously, intramuscularly, intranasally, by pulmonary aerosol or in depot form.

### DESCRIPTION OF THE FIGURES

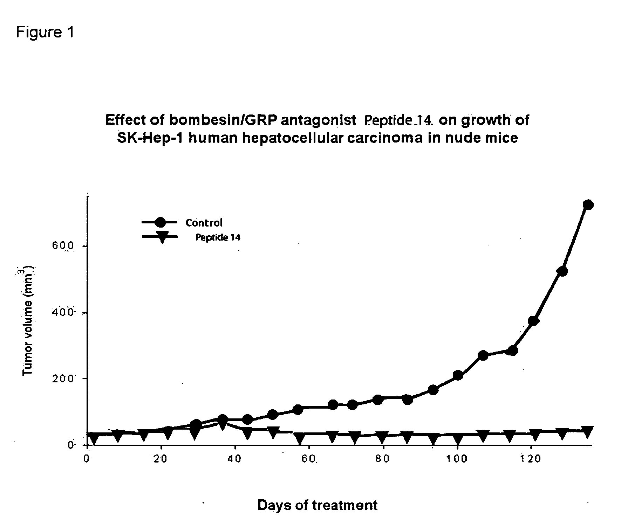

Figure 1 is a graph depicting the effect of an SK-Hep-1 human hepatocellular carcinoma in Nude mice plotting tumor volume (mm³) against days of treatment, against control, utilizing Hca¹-Gln²-Trp-Ala-Val-Gly-His-Leu-ψ-Tac⁹-NH₂ [SEQ ID NO: 7] and control, based on data drawn from Table 1.

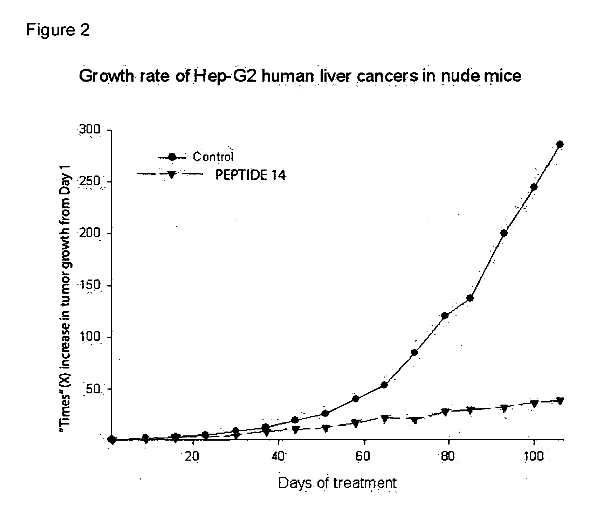

Figure 2 is a graph depicting the effect of an SK-Hep-2 human hepatocellular carcinoma in Nude mice plotting growth rate '(in "times" increase) against days of treatment, against control, utilizing Hca¹-Gln²-Trp-Ala-Val-Gly-His-Leu-ψ-Tac⁹-NH₂ [SEQ ID NO: 7], based on data drawn from Table 2.

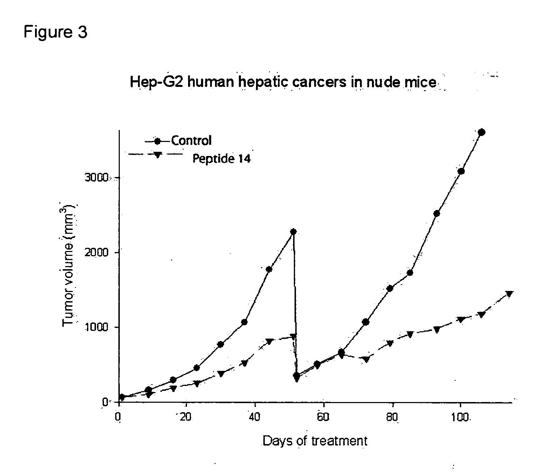

Figure 3 is a graph depicting the effect of an SK-Hep-2 human hepatocellular carcinoma in Nude mice plotting tumor volume (mm³⁾ against days of treatment, against control, utilizing Hca¹-Gln²-Trp-Ala-Val-Gly-His-Leu-ψ-Tac⁹-NH₂ [SEQ ID NO: 7] , based on data drawn from Table 1.

### DESCRIPTION OF THE PREFERRED EMBODIMENTS

### A. Synthetic Peptides 1. Nomenclature

For convenience of describing this invention, the conventional abbreviations for amino acids, peptides and their derivatives are used as generally accepted in the peptide art and as recommended by the IUPAC-IUB Commission on Biochemical Nomenclature [European J. Biochem.. 1984, 138 9-37].

The abbreviations for the individual amino acid residues are based on the trivial name of the amino acid, e.g. Ala is alanine, Cys is cysteine, Gin is glutamine, Glu is glutamic acid, pGlu is pyroglutamic acid, Gly is glycine, His is histidine, Leu is leucine, Phe is phenylalanine, Trp is tryptophan, and Val valine. Glu may have functional groups linked to its gamma carboxyl side chain: [-] or Y, defined above. Dpa is 2,3-diaminopropionic acid. Where the amino acid residue has isomeric forms, it is the L-form of the amino acid that is represented unless otherwise indicated by D-or DL- appearing before the amino acid symbol.

Abbreviations of the uncommon amino acids employed in the present invention are as follows:
Cpa is para-chlorophenylalanine
Dpa is 2,3-diaminopropionic acid pGlu is pyroglutamic acid
Nal is 3-(2-naphthyl)-alanine
Pal is 3-(3-pyridyl)-alanine
Pen is penicillamine
Tpi is 2,3,4,9 tetrahydro-1H-pyrido-[3,4-b] indole-3-carboxylic acid
Tac is thiazolidine-4-carboxylic acid
MTac is 2-Methyl-thiazolidine-4-carboxylic acid
DMTac is 5,5-Dimethyl-thiazolidine-4-carboxylic acid.

Amino acid analogues include:
Hca is hydrocinnamic acid or des-amino-phenylalanine
Hna is 3-hydroxy-2-naphthoic acid
Hpp is 3-(4-hydroxyphenyl)propionic acid
Mpp is 3-(4-methoxyphenyl)propionic acid
Naa is naphthyl acetic acid
Paa is phenylacetic acid

Other abbreviations used are:
AC acyl
Ac acetyl
AcOH acetic acid
BHA benzhydrylamine
Boc tert-butoxycarbonyl (BOC)₂O di-tert-butyldicarbonate
Bom benzyloxymethyl
But butyl
Bzl benzyl
BSA bovine serum albumin DIC 1 ,3-diisopropylcarbodiimide
DMEM Dulbecco's modified Eagle's medium
DMF dimethylformamide
Et ethyl
EDTA ethylene diamine tetraacetic acid FCBS fetal calf bovine serum
Fmoc 9-fluorenylmethyloxycarbonyl
For formyl
HITES RPMI 16 4D medium plus 10⁻⁸M hydrocortisone, 5 ul/ml bovine insulin, 10 ug/ml human transferrin, 10⁻⁸ M β-estradiol and 3 x 10⁻⁸ M Na₂SO₄
HOBt 1-hydroxybenzotriazole
HPLC high-performance-liquid-chromatography
Leu-ψ - is a reduced form of Leu where the C = O bond moiety of Leu is instead -CH₂- such that the bond of this -CH₂- moiety with the amino moiety of A⁸ is a pseudopeptide bond
Me methyl
MeCN acetonitrile
MeOH methyl alcohol
PBS phosphate-buffered saline TEA triethylamine
TFA trifluoroacetic acid

Peptide sequences are written according to the convention whereby the N-terminal amino acid is on the left and the C-terminal amino acid is on the right. It should be noted however that the convention of numbering amino acid residues in a fragmentary peptide according the corresponding position in the complete bombesin antagonist tetradecapeptide is not followed herein unless otherwise noted. If this convention were followed, the residues in the Formula I nonapeptide described herein would be numbered from 6 through 14, and the Trp- Ala-Val-Gly-His-Leu (SEQ ID NO: 13) core of the bombesin antagonists would be numbered A⁸-A⁹- A¹⁰-A¹¹-A¹²-A¹³. To avoid confusion, the bombesin antagonists are instead numbered herein as follows: the N-terminal amino acid (or amino acid analogue) residue is A¹; the C-terminal amino acid residue (or amino acid analogue) is A⁹; and the intervening residues are numbered sequentially from A² (adjacent the N- terminal residue A¹) in increasing numbers to A⁸ (adjacent the C-terminal residue A⁹).

### 2. Preferred Embodiments

The preferred embodiments are bombesin antagonist peptides of Formula I
A¹-A²-Trp-Ala-Val-Gly-His-Leu- ψ-A⁹-Q (I) (SEQ ID NO: 31)
wherein X, A¹, A², Leu-ψ-A⁹ and Q are defined as above.

These bombesin antagonist pseudopeptides are characterized by amino acid sequence, particularly at residues A¹, A² and A⁹; as well by the presence of a pseudopeptide bond between A⁸ and A⁹; and optionally by a 5-membered heterocyclic ring at A⁹. The ring structure is determined by the identity of the A⁹ residue and the compound used to oxidize it. Thus, when formaldehyde is employed and A⁹ is Cys, the resulting ring has the structure Tac of Formula II A (shown here as the Leu⁸-ψ-Tac⁹-Q fragment of the Formula I peptide where Q = NH₂

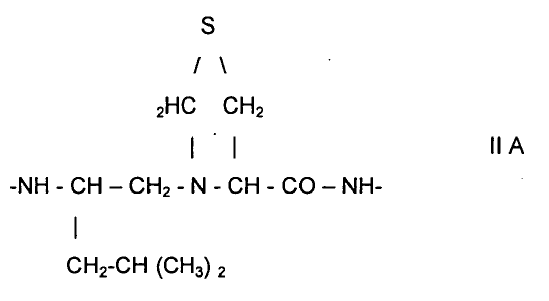

When Cys is instead oxidized with acetaldehyde, the resulting 5-membered heterocyclic ring has the structure MTac of formula IIB (shown here as the Leu⁸-ψ-MTac⁹-Q fragment of the Formula I peptide where Q = NH₂):

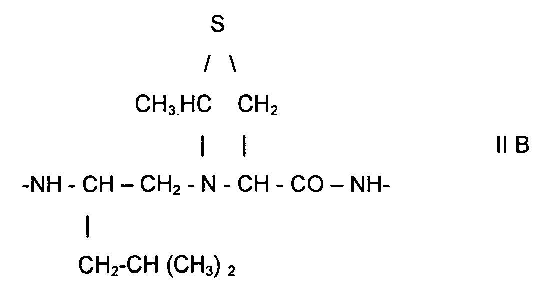

When A⁹ is instead Pen and is oxidized by formaldehyde, the resulting ring has the structure DMTac of Formula IIC (shown here as the -Leu⁸-ψ-DMTac⁹-Q fragment of the Formula I nonapeptide where Q = NH₂):

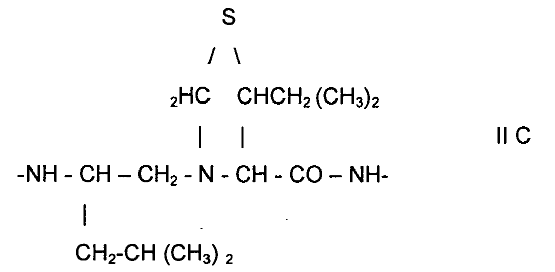

In these preferred embodiments it is desirable that X is H, Ac, or an amino acid analog, A² = Gin and Q = NH₂. Especially preferred are:
Peptide #
   1. D-pGlu-Gln-Trp-Ala-Val-Gly-His-Leu-ψ -Tac-NH₂
   2. D-Phe-Gln-Trp-Ala-Val-Gly-His-Leu-ψ -Tac-NH₂
   4. Ac-D-Phe-Gln-Trp-Ala-Val-Gly-His-Leu-ψi-Tac-NH₂
   5. D-Cpa-Gln-Trp-Ala- Val-Gly-His-Leu-ψ-Tac-NH₂
   7. D-Nal-Gln-Trp-Ala-Val-Gly-His-Leu- ψ-Tac-NH₂
   8. Pal-Gln-Trp-Ala-Val-Gly-His-Leu- ψ -Tac-NH₂ (SEQ ID NO: 14)
   9. D-Pal-Gln-Trp-Ala-Val-Gly-His-Leu-ψ-Tac-NH₂
   10. D-Trp-Gln-Trp-Ala-Val-Gly-His-Leu-ψ-Tac-NH₂
   11. Ac-D-Trp-Gln-Trp-Ala-Val-Gly-His-Leu-ψ-Tac-NH₂
   12. Tpi-Gln-Trp-Ala-Val-Gly-His-Leu-ψ-Tac-NH₂ [(SEQ ID NO: 8]
   13. D-Tpi-Gln-Trp-Ala-Val-Gly-His-Leu-ψ-Tac-NH₂
   14. Hca-Gln-Trp-Ala-Val-Gly-His-Leu-ψ-Tac-NH₂ [(SEQ ID NO: 7]
   15. D-Phe-His-Trp-Ala-Val-Gly-His-Leu-ψ-Tac-NH₂
   16. D-Phe-Glu(OMe)-Trp-Ala-Val-Gly-His-Leu-ψ-Tac-NH₂
   17. D-Phe-Glu[-]-Trp-Ala-Val-Gly-His-Leu-ψ-Tac-NH₂
   18. D-Phe-Gln-Trp-Ala-Val-Gly-His-Leu-ψ-DMTac-NH₂
   19. Ac-D-Phe-Gln-Trp-Ala-Val-Gly-His-Leu-ψ-DMTac
   20. D-Cpa-Gln-Trp-Ala-Val-Gly-His-Leu-ψ-DMTac-NH₂
   21. Tpi-Gln-Trp-Ala-Val-Gly-His-Leu-ψ-DMTac-NH₂ [(SEQ ID NO: 9]
   22. D-Tpi-Gln-Trp-Ala-Val-Gly-His-Leu-ψ-DMTac-NH₂
   23. Pal-Gln-Trp-Ala-Val-Gly-His-Leu- ψ-Cys-NH₂ [(SEQ ID NO: 10]
   24. D-Pal-Gln-Trp-Ala-Val-Gly-His-Leu-ψ-Cys-NH₂
   25. Tpi-Gln-Trp-Ala-Val-Gly-His-Leu-ψ-Cys-NH₂ [(SEQ ID NO: 11]
   26. D-Tpi-Gln-Trp-Ala-Val-Gly-His-Leu-ψ-Cys-NH₂
   27. Hca-Gln-Trp-Ala-Val-Gly-His-Leu-ψ-Cys-NH₂ [(SEQ ID NO: 12]

### B. SYNTHETIC METHODS

### 1. Overview

The pseudopeptides of Formula I may be prepared by any techniques that are known to those skilled in the peptide art. A summary of the techniques so available can be found in M. Bodanszky, Principles of Peptide Synthesis, Springer-Verlag, Heidelberg, 1984.

All the Formula I pseudopeptides may be synthesized according to the procedures of solid phase synthesis. A particularly preferred method of preparing these peptides and their intermediate peptides is solid phase synthesis. The techniques of exclusively solid-phase synthesis are set forth in the textbook of J.M. Stewart and J.D. Young, Solid Phase Peptide Synthesis, Pierce Chem. Co., Rockford, IL, 1984 (2nd. ed.) and in the review of G. Barany, et al., Int. J. Peptide Protein Res., 30, 705-739, 1987. (The additional step of oxidizing the C-terminal A⁹ residue to cyclize the residue's characteristic side chain with its alpha amino group may be performed at one of several points of synthesis of the Formula I peptides.

At least two synthetic protocols may be followed to produce the Formula I pseudopeptide bombesin antagonists. In the first protocol, all the amino acids are sequentially linked to one another after the C-terminal residue, A⁹ has been linked to a resin support phase. In the second protocol, a tripeptide is built on the resin. It is then linked to an oligopeptide carrying the remainder of the amino acid to form the bombesin antagonist. In both protocols, synthesis begins at the C- terminal A⁹ residue and adds amino acid residues sequentially with growth toward the N-terminal residue.
1. (a) First Protocol. The resin support phase employed in the solid phase synthesis of the pseudopeptides may be benzhydrylamine (BHA) resin or chloromethylated polystyrene resin 1 % crosslinked with divinylbenzene, which are both commercially available. The C-terminal A⁹ residue is attached to this resin via its carboxyl group. Reaction between two of these residues is prevented by attaching a chemical protecting group at the alpha amino group of each A⁹ residue prior to linking it to the resin. The protecting group for the alpha amino group of the A⁹ residue, and each subsequently linked amino acid residue, may be either 9-fluorenylmethyloxycarbonyl (Fmoc) or tert-butoxycarbonyl (Boc) group. Fmoc is preferred in coupling the C-terminal residue A⁹ through A² since the reaction removing Boc also removes the side chain protecting group from the C-terminal A⁹ residue. During these linking reactions, the characteristic side chain functional groups of certain amino acid residues may also be protected from undesired chemical reaction by attaching a chemical protecting group thereto (suitably But for A⁹) prior to the linkage reaction.
   After the C-terminal Fmoc-A⁹(But) residue is joined to the support phase, its alpha amino group is deprotected and Fmoc-Leu-CHO is coupled thereto, thus forming the pseudopeptide bond.
   The remaining residues, A^{B}, A⁴, A³ and A², suitably protected by Fmoc, and A¹ suitably protected by Boc, are added step-wise in reverse numerical order (A⁷, A^{β}, etc.) to construct the desired pseudopeptide.
   When His or Glu is present in the pseudopeptide, their side chains may be vulnerable to undesirable chemical reactions during their linking reactions or the linking reactions of other residues. Accordingly, a chemical protecting group may be attached to such side chains, prior to linking these amino acids in the linking reaction.
   Once all the amino acid residues are linked to the BHA resin, the pseudo¬ peptide's Boc at the N-terminal may be removed. The Tac, MTac or DMTac 5-membered heterocyclic ring is formed by reaction of the exposed -CH₂-SH moiety of Cys (or the -C(CH₃)₂SH of Pen) and the secondary amino of the reduced bond joining residues A⁸ and A⁹ (i.e., the alpha amino of A⁹) with an oxidizing reagent, such as formaldehyde or acetaldehyde. The intermediate pseudopeptide, still bearing side chain protecting groups, is subjected to HF treatment to cleave it from the resin support phase and also to remove the side chain protecting groups.
1(b) Second Protocol.
   In the second protocol, following solid phase synthesis pathways, the tripeptide is built up on the BHA resin to the protected tripeptide resin:
   Boc-His(Bom)⁷-Leu⁸-ψ -Cys(But)⁹-BHA resin.

The Boc¹ group and the But group are removed and the -CH₂-SH moiety of Cys is cyclized with the secondary amine of the reduced bond joining residues A⁸ and A⁹ to get His (Bom)⁷-Leu⁸-psi-Tac⁹-BHA resin. After HF treatment, the free tripeptide His⁷-Leu⁸-psi-Tac⁹-NH₂ is obtained. Boc A¹-A²-Trp-Ata-Val-Gly-OCH₂- resin is built step by step on Gly-OCH₂-resin in accordance with the standard methods of solid phase synthesis, and then treated in 95% methanol containing 1 % KCN for 12 hours to cleave the Boc-oligopeptide. After the two fragments are constructed, the Boc-oligopeptide is linked to the free His⁷-Leu⁸-ψ -Tac⁹-NH₂ tripeptide with BOP reagent. The Boc group is then removed to yield the desired peptide.

Before describing synthesis of the Formula I bombesin antagonists in detail, several synthetic Operations common to one or both of the above protocols are described.

### 2. General Operations for Polypeptide Synthesis

### Operation 1: Formation of certain synthetic residues or reactants.

a) L- and D-Tpi:
   2.04 g (10 mM) of L-Trp is dissolved in 25 ml of boiling water containing 2.1 g of citric acid. 0.5 ml 40% aqueous formaldehyde are added and solids begins to form immediately. The mixture is chilled in an ice bath and the precipitates collected and washed with cold water and air, then dried at room temperature to yield 2.14 g or 99% solids m.p. with (decomposition) ca. 310°. The D-isomer is formed in the same manner from D-Trp and also has m.p. (decomposition) ca. 310°C.
b) L- and D-Boc-Tpi:
   To a stirred suspension of 10.8 g (50 mM) of D-Tpi in 250 ml of 0.2 N NaOH and 7.5 ml triethyl amine was added 10g of Di-tert- butyl dicarbonate, the mixture stirred 4 hrs then another 10 g of dicarbonate added and a further 10g after another 3 hrs. of stirring. The mixture is stirred overnight and extracted (2x 100 ml) with ether, which is discarded. Citric acid was added to the aqueous layer to reach a pH of 3-5. The solids are collected, washed with water and air dried overnight.
   The solids are suspended in 100 ml tetrahydrofuran. Almost all solids dissolved. The insolubles are removed by filtration and THF removed under vacuum. The residue is triturated with ether to yield 9.20 g or 58%. This material has same m.p. as the starting material, but differs in solubility and TLC on silica using 85:15:0.5 CHCl₃:MeOH:HOAc.
   2.55 g of L-Tpi gives 2.22 g or 59% of Boc-Tpi using the same method.
c) Fmoc-Leu-CHO
   Fmoc-Leucine methyl ester (35 g, 134 mmoles) in dry toluene (250 ml) under N₂ is cooled with dry ice/acetone, and 150 ml of 25% di-isobutyl-aluminum hydride in toluene are added over 30 mins. The mixture is stirred for 20 min in a bath of dry ice/acetone after the addition of the di-isobutyl aluminum hydride, then methanol (15 ml) is added cautiously. The mixture is poured into 1000 ml ice-cold water, shaken and filtered. The toluene is separated and the aqueous phase re-extracted with ether (3x300 ml). Toluene and ether extracts are combined and dried over Na₂SO₄. The resulting oil is passed rapidly though a silica gel column (3x50 cm) in 1500 ml 15% EtOAc/petrol. The Fmoc-Leu-CHO is obtained as a solid (27.6g).
d) Synthetic amino acids or amino acid analogs to be incorporated in the bombesin antagonist pseudopeptides are generally available from commercial sources. Thus, Hca is commercially available from Aldrich Co., 1001 St. Paul Avenue, Milwaukee, WI 53233. Boc- or Fmoc-protected amino acids are available commercially from Advanced ChemTech, 5609 Fern Valley Road, Louisville, KY 40228; or Bachem California, 3132 Kashiwa Street, Torrance, CA 90505.

### Operation 2: Preparation of resin and addition of A⁹ residue.

The BHA resin is prepared by treatment with 10% TEA in CH₂Cl₂(neutralization) twice each for three minutes and washed with CH₂Cl₂ six times. The Fmoc-A⁹(But) residue is bound to the resin by adding with 1 .35 mmole Fmoc- A⁹(But) and 1.50 mmoles 1-hydroxy-benzotriazole (HOBt) in DMF and mixing for three minutes. 20% 1,3-diisopropyl-carbodiimide (DIC) with 1.3 mmoles in CH₂Cl₂ is added. The mixture is shaken at room temperature for 60 minutes. The resulting Fmoc-A⁹(But)-BHA resin is washed with CH₂Cl₂, methanol two times each, and CH₂Cl₂ three times, and then subjected to a Kaiser test (Anal. Biochem. 34. 595 (1970)). In case of incomplete coupling, the procedure is repeated.

### Operation 3: Formation of pseudopeptide bond

The deprotection of Fmoc group from A⁹ is performed by adding 50% piperidine in DMF and mixing for 30 min; washing with DMF (6 x 1 min); then i) adding Fmoc-Leu-CHO (3 equiv.) in DMF containing 1 % AcOH; and ii) adding NaBH₃CN (3.5 equiv.) in DMF and shaking 60 min. This is followed by washing with 50% MeOH (3 x 1 min); 100% MeOH (3 x 1 min); and DMF (3 x 1 min).

### Operation 4: Coupling of amino acids and Formation of peptide bond

The protecting group for the alpha amino group of the A⁹ residue, and each subsequently linked amino acid residue, may be either 9-fluorenylmethyioxycarbonyl (Fmoc) or tert-butoxycarbonyl (Boc) group. Fmoc is preferred in coupling A⁹ through the amino acid residue just adjacent the N-terminal amino acid residue-since the reaction removing Boc also removes the side chain protecting group of A⁹.

### A) Using Fmoc-amino acid

The following procedures are performed for introducing an Fmoc amino acid to an Fmoc-intermediate peptide.
(1) The Fmoc-intermediate peptide is deprotected and neutralized, then washed with CH₂Cl₂ (3 x 1 min) and DMF (3 x 1 min).
(2) The Fmoc-amino acid is coupled to the deprotected intermediate peptide by: i) adding Fmoc amino acid (3 equiv.) and HOBt (3.3 equiv.) in DMF (3 min) to the intermediate peptide; ii) adding 3 equiv. DIC (as 20% solution in CH₂Cl₂) and shaking the mixture for 90 min.
(3) The mixture is then washed with ethanol (3 x 1 min) and DMF (3 x 1 min).

In order to couple further residues, the newly coupled Fmoc-amino acid is deprotected with 50% piperidine in DMF for 30 min, and washed with DMF (6 x 1 min). Other couplings follow as described at step (2).

Each time a new amino acid is coupled to the resin or peptide, a Kaiser test is performed; in case of incomplete coupling occurs, the reaction is repeated.

For the coupling of Fmoc-Gly and Fmoc-Gln, step (2) of this Operation is modified as follows: 3 equiv. DIC (as 20% CH₂Cl₂) is added to a mixture DMF solution of Fmoc amino acid (3.0 equiv.) and HOBt (3.3 equiv.) at 0°C for 15 min and at room temperature 15. The reaction mixture is added to peptide resin, shaken for 1 hour if Fmoc-Gly and for 2 hours if Fmoc-Gln.

### B) Using Boc-amino acid

The following procedures are performed for introducing a Boc-amino acid to an Fmoc-intermediate peptide.
(1)The Boc group is removed by adding 50% TFA in CH₂Cl₂;
(2) adding 50% TFA in CH₂Cl₂ containing 5% mercaptoethanol and 5% anisole for 25 minutes; and
(3) washing with CH₂Cl₂ (2 times 1 min), MeOH (2 times 1 min), and DMF (3 times 1 min).
(4) Coupling of the Boc-amino acid residue is performed by adding 3 equivalents of Boc-amino acid and 3.3 equivalents HOBt in DMF and mixing for 3 minutes. Then 3 equivalents of 20% diisopropylcarbodiimide in CH₂Cl₂ are added and shaken for 90 minutes. The reaction mixture is then washed with MeOH (3 times 1 minute) and then CH₂Cl₂ (3 times 1 minute).
(5) The Boc group is removed (deprotection) by washing the product of step (5) with 50% TFA in CH₂Cl₂ containing 5% mercaptoethanol (5 minutes) and 5% anisole (25 minutes). Further washes in CH₂Cl₂ (2 times 1 minute), MeOH (2 times 1 minute) and DMF (3 times 1 minute) follow.

It should be kept in mind that removal of the Boc group also removes the But protecting group suitably coupled to the A⁹ side chain. Thus, removal of Boc is usually not performed until just before cyclization of the A⁹ residue.

### Operation 5: Formation of 5-membered heterocyclic ring

The ring structure of Formula IIA is formed by shaking the intermediate peptide having deprotected Cys⁹ in a mixture of 50% AcOH, 3.7% HCHO and DMF (1 :1 :8) at room temperature for 3 minutes and filtered. The product is washed with DMF, MeOH and CH₂Cl₂ three times each.

The ring structure of Formula IIB is formed by shaking the intermediate with peptide having deprotected Cys⁹ in a mixture of 50% AcOH, 10% CH₃CHO and DMF (1.5:0.5:8) at room temperature for 10 minutes and filtered. The product is washed with DMF, MeOH and CH₂Cl₂ three times each.

The ring structure of Formula IIC is formed by shaking the intermediate with peptide having deprotected Pen⁹ in a mixture of 50% AcOH, 3.7% HCHO and DMF (1 :1 :8) at room temperature for 10 minutes and filtered. The product is washed with DMF, MeOH and CH₂Cl₂ three times each.

### Operation 6: Detachment of peptide from resin

After all the desired amino acid residues have been added, the intermediate peptide resin is treated with liquid HF in the presence of anisole to cleave the polypeptide from the support phase. This reaction removes side chain protecting groups as well. When BHA resin is used, the resulting intermediate peptide has Q = NH₂.

### Operation 7: Purification of Peptides

The pseudopeptides of Formula I are generally purified by high performance liquid chromatography (HPLC) on a reversed phase column carried out on a Rainin HPLC System (Rainin Inc., Co., Woburn, MA) consisting of three Rainin Rabbit HP HPLC pumps controlled by an Apple Macintosh Plus™ computer, a Rheodyne™ Injector and a Knauer™ Model 87 variable wavelength UV monitor. Crude peptides (10-40 mg) are loaded on a Dynamax™ Macro column (21.2 X 250 mm) packed with spherical C₁₈ silica gel (pore size: 300A; particle size: 12 µm) (Rainin Inc. Co.) and eluted with linear gradient by using a solvent system consisting of (A) 0.1 % TFA and (B) 0.1 % TFA in 70% aqueous acetonitrile at a flow rate of 2.0 ml/min. All fractions are assessed for purity and retention time by an Analytical HPLC described at below.

The quality and the elution characteristics of crude and purified peptide are established by analytical HPLC on a Hewlett-Packard™ Model 1090 liquid chromatography equipped with a diode array detector set at 220 and 280 nm and a reversed phase 4.6 X 250 mm W-porex™ C₁₈ column (pore size: 300A°, particle size: 5 µm). A flow rate of 1.2 ml/min of solvent system (A) and (B) described as above is maintained and the separations were performed at room temperature.

In most cases, pseudopeptide bombesin antagonists were further purified by rechromatography on the same column with slight modification to the gradient conditions. The homogeneity of purified peptides proved to be pure over 97% in analytical HPLC.

If desired, amino acid analysis of the pseudopeptides of Formula I may be performed in a Beckmann 6300 amino acid analyzer, on samples hydrolyzed at 1 10°C for 20 hrs. in sealed, evacuated tubes with 4 M methanesulfonic acid containing 0.2% 3-(2-aminoethyl)-indole.

### C. SYNTHETIC INTERMEDIATES

### 1.Side Chain Protecting Groups

In solid phase synthesis, it is common to protect reactive side chain functional groups of the various amino acid moieties or peptide fragments. These side chain functional groups may be protected in order to prevent an undesirable chemical reaction from occurring at said chains. Accordingly, it is common that an intermediate peptide is produced which includes each of the amino acyl residues located in its desired sequence in the peptide chain with side-chain protecting groups linked to the appropriate residues.

In selecting a particular side chain protecting group to be used in the synthesis of the peptides, these rules are generally followed: (a) the protecting group preferably retains its protecting properties under coupling conditions, (b) the protecting group should be stable to the coupling reagents, is preferably stable under the coupling reaction conditions selected for removing the alpha amino protecting group at each step of the synthesis and, (c) the side chain protecting group must be removable upon the completion of the synthesis of the desired amino acid sequence, under reaction conditions that will not undesirably alter the peptide chain.

The side chain protecting groups are attached to the amino acid residues by steps well known in the art. Suitable side chain protecting groups for His include Bom; and for Glu and Cys include But.

### 2.Synthetic Intermediates

Also considered to be within the scope of the present invention are intermediate peptides at the following stages of synthesis. Illustrated here are intermediate peptides of Peptide 2 at three stages:
Stage A: following linkage of all the amino acids and the resin:
   Boc-D-Phe-Gln-Trp-Ala-Val-Gly-His(Bom)-Leu-ψ-Cys(But)-BHA resin(1 /02/A");
Stage B: following removal of the N-terminal Boc-group:
   D-Phe-Gln-Trp-Ala-Val-Gly-His(Bom)-Leu-ψ-Cys-BHA resin ("1/02/B");
Stage C: following cyclization of A⁹:
   D-Phe-Gln-Trp-Ala-Val-Gly-His(Bom)-Leu-ψ-Tac-BHA resin ("1/02/C").

There is one further stage, Stage D, illustrated in the synthesis of Peptide No. 17 in Example 2 below. The Stage D intermediate is removed from the resin but does not yet have the single bond X, linking A¹ to A².

### MEDICAL APPLICATIONS

Since these compounds of this invention are antagonists of bombesin/GRP receptors, they can be used in treatment of human hepatomas.

The Formula I bombesin antagonists of the invention may be administered in the form of pharmaceutically acceptable nontoxic acids or salts, such as acid addition salts. Illustrative of such acid addition salts are hydrochloride, hydrobromide, sulphate, phosphate, fumarate, gluconate, tannate, maleate, acetate, citrate, benzoate, succinate, alginate, pamoate, malate, ascorbate, tartrate, and the like.

These pharmaceutical compositions will contain the Formula I pseudo¬ peptides in conjunction with a conventional, pharmaceutically-acceptable carrier, e.g., physiological saline being acceptable, though other carriers known to the art may be used.

Treatment of subjects with these pharmaceutical compositions may be carried out in the same manner as the clinical treatment using other agonists and antagonists of LHRH, or somatostatin analogues. Thus, the Formula I bombesin antagonists may be administered intravenously, subcutaneously, intramuscularly, intranasally or by pulmonary aerosol or in a depot form (eg. microcapsules, microgranules or cylindrical rod-like implants) formulated from a biodegradable suitable polymer (such as DL-lactide-coglycolide). Other equivalent modes of administration are also within the scope of this invention, i.e, continuous drip, infusion pump and time-release modes, such as microcapsules and the like.

Effective dosages of Formula I peptides will vary with the form of administration and the particular species of mammal being treated. The dosage will be from about 1 to 1000 micrograms of the peptide per kilogram of the body weight of the host per day when given parenterally. These dosage ranges are merely preferred and higher dosages may be chosen in appropriate circumstances. A physiological saline solution containing the peptide may be administered to provide a dose in the range of about 0.01 to 0.20 mg/kg of body weight per day, except for depot forms where the amount injected is to be calculated to last from about 15 to about 30 days or longer.

### EXAMPLES

In the following Examples, a three character code is utilized to identify intermediate peptides at certain stages of synthesis. A peptide encoded "1/01 /A" is the intermediate peptide for Peptide "01 " made in Example " 1 " which has completed Stage A in synthesis. Similarly, "2/08/B" and "4/19/C" are inter- mediate peptides of Peptides "08" and "19" in Examples 2 and 4 which have completed synthesis Stages B and C respectively.

### Example (1 )

### Peptide #

1. D-pGlu-Gln-Trp-Ala-Val-Gly-His-Leu-ψ -Tac-NH₂
2. D-Phe-Gln-Trp-Ala-Val-Gly-His-Leu-ψ -Tac-NH₂
4. Ac-D-Phe-Gln-Trp-Ala-Val-Gly-His-Leu-ψ-Tac-NH₂
5. D-Cpa-Gln-Trp-Ala- Val-Gly-His-Leu-ψ-Tac-NH₂
7. D-Nal-Gln-Trp-Ala-Val-Gly-His-Leu- ψ -Tac-NH₂
8. Pal-Gln-Trp-Ala-Val-Gly-His-Leu- ψ -Tac-NH₂ (SEQ ID NO: 15)
9. D-Pal-Gln-Trp-Ala-Val-Gly-His-Leu-ψ-Tac-NH₂
10. D-Trp-Gln-Trp-Ala-Val-Gly-His-Leu-ψ-Tac-NH₂
11. Ac-D-Trp-Gln-Trp-Ala-Val-Gly-His-Leu-ψ-Tac-NH₂
12. Tpi-Gln-Trp-Ala-Val-Gly-His-Leu-ψ-Tac-NH₂ [(SEQ ID NO: 8]
13. D-Tpi-Gln-Trp-Ala-Val-Gly-His-Leu-ψ-Tac-NH₂
14. Hca-Gln-Trp-Ala-Val-Gly-His-Leu-ψ-Tac-NH₂ [(SEQ ID NO: 7]

These peptides may suitably be synthesized from a common intermediate 1-1 , Fmoc-Trp-Ala-Val-Gly-His(Bom)-Leu- ψ -Cys(But)-BHA (SEQ ID NO: 16) resin, and are synthesized as follows.

Fmoc-Leu- ψ -Cys(But)-BHA resin is obtained as follows: 2.0 g BHA resin (0.55 mmol NH₂/g) is prepared by treatment with 20ml 10% TEA in CH₂Cl₂ (neutralization) twice each for 3 minutes and washed with 20 ml CH₂Cl₂ six times; then mixed with 3.3 mmole Fmoc-Cys(But) and 3.6 mmole HOBt in DMF for three minutes. 3 equiv. DIC (as 20% solution in CH₂Cl₂) is added. The mixture is shaken at room temperature for 90 minutes. The resulting Fmoc-Cys(But)-BHA resin is washed with CH₂Cl₂, methanol two times each, and CH₂Cl₂ three times, and then subjected to a Kaiser test.

The coupling of Fmoc-Leu-CHO to form the pseudopeptide bond is performed as follows. The deprotection of Fmoc group from A⁹ is performed by adding 15 ml 50% piperidine in DMF and mixing for 30 min and washing with 15 ml DMF (6 x 1 min). Then 3.3 mmole Fmoc-Leu-CHO (3 equiv.) in DMF containing 1 % AcOH is added, followed by NaBH₃CN (3.5 equiv.) in DMF and shaking 1 hour. This is followed by washing with 15 ml 50% MeOH (3 x 1 min); 15 ml 100% MeOH (3 x 1 min); and 15 ml DMF (3 x 1 min).

The removal of the Fmoc-group (deprotection) from Fmoc-Leu-ψ-Cys(But)-BHA resin is carried out per Operation 4A.

After the removal of the Fmoc group from Fmoc-Leu-ψ-Cys(But)-BHA resin and neutralization, the coupling of Fmoc-His(Bom) is carried out as described in Operation 4A.

The coupling of Fmoc-Gly is performed as in Operation 4. 20% 1 ,3-diisopropylcarbodiimide (3.3 mmole) in CH₂Cl₂ was added to a DMF solution of 3.3 mmoles Fmoc-Gly and 3.6 mmoles HOBt at 0°C, stirred under cooling for 15 min and at room temperature for 15 min, the precipitate filtered off and added to resin, and shaken for 60 min. The subsequent amino acid residues Fmoc-Val, Fmoc-Ala and Fmoc-Trp are then sequentially introduced by coupling in the same manner to yield 3.80 g of intermediate 1-1 , the protected peptide resin with structure Fmoc-Trp-Ala-Val-Gly-His(Bom)-Leu-ψ-Cys(But)-BHA (SEQ ID NO: 16) resin.
Sequential coupling of Fmoc-Gln and Boc-D-pGlu to intermediate peptide 1-1 yields: Boc-D-pGlu-Gln-Trp-Ala-Val-Gly-His(Bom)-Leu-ψ-Cys(But)-BHA resin ("1/01 /A").
Sequential coupling of Fmoc-Gln and Boc-D-Phe to intermediate peptide 1-1 yields: Boc-D-Phe-Gln-Trp-Ala-Val-Gly-His(Bom)-Leu-ψ-Cys(But)-BHA resin ("1/02/A").
Sequential coupling of Fmoc-Gln and Ac-D-Phe to intermediate peptide 1-1 yields: Ac-D-Phe-Gln-Trp-Ala-Val-Gly-His(Bom)-Leu-ψ-Cys(But)-BHA resin ("1 /04/A").
Sequential coupling of Fmoc-Gln and Boc-D-Cpa to intermediate peptide 1-1 yields: Boc-D-Cpa-Gln-Trp-Ala-Val-Gly-His(Bom)-Leu-ψ-Cys(But)-BHA resin
Sequential coupling of Fmoc-Gln and Boc-D-Nal to intermediate peptide 1-1 yields: Boc-D-Nal-Gln-Trp-Ala-Val-Gly-His(Bom)-Leu-ψ-Cys(But)-BHA resin ("1/07/A").
Sequential coupling of Fmoc-Gln and Boc-Pal to intermediate peptide 1-1 yields: Boc-Pal-Gln-Trp-Ala-Val-Gly-His(Bom)-Leu-ψ-Cys(But)-BHA (SEQ ID NO: 17) resin ("1 /08/A").
Sequential coupling of Fmoc-Gln and Boc-D-Pal to intermediate peptide 1-1 yields: Boc-D-Pal-Gln-Trp-Ala-Val-Gly-His(Bom)-Leu-ψ-Cys(But)-BHA resin ("1/09/A").
Sequential coupling of Fmoc-Gln and Boc-D-Trp to intermediate peptide 1-1 yields: Boc-D-Trp-Gln-Trp-Ala-Val-Gly-His(Bom)-Leu-ψ-Cys(But)-BHA resin ("1/10/A").
Sequential coupling of Fmoc-Gln and Ac-D-Trp to intermediate peptide 1-1 yields: Ac-D-Trp-Gln-Trp-Ala-Val-Gly-His(Bom)-Leu-ψ-Cys(But)-BHA resin ("1/11 /A").
Sequential coupling of Fmoc-Gln and Boc-Tpi to intermediate peptide 1-1 yields: Boc-Tpi-Gln-Trp-Ala-Val-Gly-His(Bom)-Leu-ψ-Cys(But)-BHA (SEQ ID NO: 18) resin ("1/12/A").
Sequential coupling of Fmoc-Gln and Boc-D-Tpi to intermediate peptide 1-1 yields: Boc-D-Tpi-Gln-Trp-Ala-Val-Gly-His(Bom)-Leu-ψ-Cys(But)-BHA resin ("1/13/A").
Sequential coupling of Fmoc-Gln and Hca to intermediate peptide 1-1 yields: Hca- Gln-Trp-Ala-Val-Gly-His(Bom)-Leu-ψ-Cys(But)-BHA (SEQ ID NO: 19) resin ("1/14/A").

The N-terminal Boc group of intermediate peptide 1/01/A is then removed by treatment with 50% TFA in CH₂Cl₂ containing 5% mercaptoethanol and 5% anisole twice, first for 5 minutes and then for 25 minutes. The intermediate peptide is then washed with CH₂Cl₂ (2 times 1 min), MeOH (2 times 1 min) and DMF (3 times 1 min). This also removes the But protecting group from Cys, producing intermediate peptide D-pGlu-Gln-Trp-Ala-Val-Gly-His(Bom)-Leu-ψ-Cys-BHA resin ("1/01/B"), which is then washed with CH₂Cl₂, MeOH and DMF, three times each for one minute.

At this point, the Cys side chain of intermediate peptide 1/01 /B is cyclized by oxidation to form the 5-membered heterocyclic ring shown in Formula IIA per Operation 5. A 10 ml mixture of AcOH, 3.7% HCHO and DMF (1:1:8) is added. The reaction mixture is shaken at room temperature for 3 minutes, washed with water, DMF and CH₂Cl₂ 3 times each to yield intermediate peptide 1 /01 /C, D- pGlu-Gln-Trp-Ala-Val-Gly-His(Bom)-Leu-ψ-Tac-BHA resin.

Intermediate peptide 1/01/C is then treated with HF and anisole per Operation 6 to remove the Bom protecting group from His and simultaneously cleave the nonapeptide from the resin support phase resulting in a C-terminal Q group which is-NH₂. The peptide is purified with HPLC per Operation 7. The bombesin antagonist peptide number 1 is thus obtained.

The same steps of removing the Boc group and But group and cyclizing the-CH₂-SH group of Cys with the secondary amine of the reduced bond are performed on intermediate peptides 1/02/A, 1/05/A, 1/07/1A, 1/08/A, 1/09/A, 1/10/A and 1/12/A to yield intermediate peptides as follows:
1/02/B D-Phe-Gln-Trp-Ala-Val-Gly-His(Bom)-Leu-ψ-Tac-BHA resin
1/04/B Ac-D-Phe-Gln-Trp-Ala-Val-Gly-His(Bom)-Leu-ψ-Tac-BHA resin 1/05/B
D-Cpa-Gln-Trp-Ala-Val-Gly-His(Bom)-Leu-ψ-Tac-BHA resin
1/07/B D-Nal-Gln-Trp-Ala-Val-Gly-His(Bom)-Leu-ψ-Tac-BHA resin
1/08/B Pal-Gln-Trp-Ala-Val-Gly-His(Bom)-Leu-ψ- Tac-BHA resin (SEQ ID NO: 20)
1/09/B D-Pal-Gln-Trp-Ala-Val-Gly-His(Bom)-Leu-ψ-Tac-BHA resin
1/10/B D-Trp-Gln-Trp-Ala-Val-Gly-His(Bom)-Leu-ψ-Tac-BHA resin
1/11/B Ac-D-Trp-Gln-Trp-Ala-Val-Gly-His(Bom)-Leu-ψ-Tac-BHA resin
1/12/B Tρi-Gin-Trp-Ala-Val-Gly-His(Bom)-Leu-ψ-Tac-BHA resin (SEQ ID NO: 21)
1/13/B D-Tpi-Gln-Trp-Ala-Val-Gly-His(Bom)-Leu-ψ-Tac-BHA resin
1/14/B HCa-Gln-Trp-Ala-Val-Gly-His(Bom)-Leu-ψ-Tac-BHA resin (SEQ ID NO: 22)

The crude peptides are cleaved from BHA resin, and then purified by Operation 6 and 7 to yield pure peptides 2, 4, 5, and 7-14.

The retention time of these peptides is as follows.

### Analytical HPLC data

| Gradient %B/min | Retention time on Column D |
|---|---|
| 25-65 | 14.40 |
| 25-65 | 14.20 |
| 25-75 | 15.17 |
| 25-65 | 16.11 |
| 20-60 | 23.75 |
| 20-60 | 13.19 |
| 25-65 | 12.00 |
| 25-65 | 16.98 |
| 25-65 | 24.50 |
| 25-65 | 19.91 |
| 25-65 | 16.99 |
| 40-80 | 13.68 |

Alternatively, the 5-membered heterocyclic ring may be formed in a solution with the procedure as follows:

25 mg of an intermediate peptide including the structure Leu-ψ-Cys-NH₂ obtained from solid phase synthesis in 0.8 ml glacial acetic acid is mixed with 100 ul 1 % formaldehyde (wt % solution in water) at room temperature for 30 seconds, and then 20 ul 50% ammonium acetate solution is added. The reaction mixture is subjected purification to yield the desired peptide which contains the structure -Leu-ψ-Tac-NH₂.

### Example (2)

### Peptide #

15. D-Phe-His-Trp-Ala-Val-Gly-His-Leu-ψ-Tac-NH₂
16. D-Phe-Glu(OMe)-Trp-Ala-Val-Gly-His-Leu-ψ-Tac-NH₂
17. D-Phe-Glu[-]-Trp-Ala-Val-Gly-His-Leu-ψ-Tac-NH₂

The peptides in this example may suitably be synthesized from the common intermediate 1-2, Fmoc-Trp-Ala-Val-Gly-His(Bom)-Leu-ψ-Cys(But)-BHA (SEQ ID NO: 16) resin, built step by step on 0.5g BHA resin (0.55 mmoles NH₂/g). Intermediate peptide 1-2 is prepared according to the Operations and procedures set out in Example (1 ). A 150 mg portion of intermediate 1-2 is then divided into 3 aliquots and subjected to two further couplings with the procedure described in Operation 4 to yield the final nonapeptide resins.
Sequential coupling of Fmoc-His(Bom) and Boc-D-Phe to intermediate peptide 1-2 yields:
   Boc-D-Phe-His(Bom)-Trp-Ala-Val-Gly-His(Bom)-Leu-ψ-Cys(But)-BHA resin ("2/15/A").
Sequential coupling of Fmoc-Glu(OMe) and Boc-D-Phe to intermediate peptide 1-2 yields:
   Boc-D-Phe-Glu(OMe)-Trp-Ala-Val-Gly-His(Bom)-Leu-ψ-Cys(But)-BHA resin ("2/16/A").
Sequential coupling of Fmoc-Glu(But) and Boc-D-Phe to intermediate peptide 1-2 yields:
   Boc-D-Phe-Glu(But)-Trp-Ala-Val-Gly-His(Bom)-Leu-ψ-Cys(But)-BHA resin ("2/17/A").

The removal of Boc-group from intermediate peptide 2/15/A is performed using 50% TFA in CH₂Cl₂ containing 5% mercaptoethanol and 5% anisole per Operation 4. This reaction also removes the But group from residue A⁹, resulting in intermediate peptide 2/15/B:
D-Phe-His(Bom)-Trp-Ala-Val-Gly-His(Bom)-Leu-ψ-Cys-BHA resin.

At this point, the Cys side chain of intermediate peptide 2/15/B is cyclized by oxidation to form the 5-membered heterocyclic ring shown in Formula HA per Operation 5. A 10 ml mixture of AcOH, 3.7% HCHO and DMF (1 :1 :8) is added. The reaction mixture is shaken at room temperature for 3 minutes, washed with water, DMF and CH₂Cl₂ 3 times each to yield intermediate peptide 2/15/C:
D-Phe-His(Bom)-Trp-Ala-Val-Gly-His(Bom)-Leu- ψ -Tac-BHA resin.

Then, intermediate peptide 2/15/C is treated with freshly distilled HF (5 ml) and anisole (0.25 ml) at 0°C for 1 hour, thereby also removing the Bom protecting group from His. The solvent is evaporated in vacuo and washed with ethyl acetate, extracted with 70-80% aqueous acetic acid and lyophilized. After purification, bombesin antagonist peptide number 15 is obtained.

The same steps to remove the N-terminal Boc, cyclize Cys⁹, remove the intermediate peptide from BHA resin and purify with HPLC are performed on intermediate peptides 2/16/A and 2/17/A to yield peptide number 16 and intermediate peptide 2/17/D:
D-Phe-Glu-Trp-Ala-Val-Gly-His-Leu- ψ-Tac-NH₂.

A mixture of 15 mg of 2/17/D, and 15 mg HOBt in 0.8 ml DMF at 0°C Is added to 50 microliters 25% diisopropyl carbodiimide in CH₂Cl₂ and stirred at 0°C for 2 hours. The single bond of peptide 17 linking the alpha amino of D-Phe¹ to the gamma carboxyl moiety on the 3-propionyl moiety of Glu² is formed:
D-Phe-Glu[-]-Trp-Ala-Val-Gly-His-Leu-ψ-Tac-NH₂

The reaction mixture is subjected to purification with HPLC in accordance with Operation 7.

The retention times of these peptides are as follows. Analytical HPLC data

| Peptide No. | Gradient %B/min | Retention time on Column D |
|---|---|---|
| 15 | 20-60 | 17.23 |
| 16 | 25-65 | 19.13 |
| 17 | 20-60 | 20.34 |

### Example (3)

### Peptide #

3. D-Phe-Gln-Trp-Ala-Val-Gly-His-Leu-ψ-MTac-NH₂
6. D-Cpa-Gln-Trp-Ala-Val-Gly-His-Leu-ψ-MTac-NH₂

These bombesin antagonists may suitably be synthesized from a common intermediate 1-3, i.e., Fmoc-Gln-Trp-Ala-Val-Gly-His(Bom)-Leu-ψ-Cys(But)-BHA (SEQ ID NO: 23) resin. This is built on 1.0 g. BHA resin (0.55m moles NH₂/g) by the successive coupling with solid phase synthesis operations as described at Example (1 ) beginning with Fmoc-Cys(But) and followed by Fmoc-Leu-CHO (with NaBH₃CN); and Fmoc-His(Bom), etc. further according to Example (1 ). Fmoc-Gln is linked to the N terminal according to Operation 4 to form the Intermediate I-3. The final coupling of Boc-D-Phe or Boc-D-Cpa to Intermediate I-3, is performed according to the procedure in Operation 4 to form intermediate peptides "3/3/A" or "3/6/A" respectively.

The removal of Boc group is performed using 50% TFA in CH₂Cl₂ containing 5% mercaptoethanol and 5% anisole per Operation 4.

At this point, the Cys side chain of intermediate peptide 3/3/B is cyclized by oxidation to form the 5-membered heterocyclic ring shown in Formula IIB per Operation 5. A 10 ml mixture of 50% AcOH, 10% CH₃CHO and DMF (1.5:0.5:8) is added. The reaction mixture is shaken at room temperature for 10 minutes, washed with water, DMF and CH₂Cl₂ 3 times each to yield intermediate peptide 3/3/C: D-Phe-Gln-Trp-Ala-Val-Gly-His-Leu-ψ-MTac-BHA resin.

Intermediate peptides 3/3/C and 3/6/C are removed from the resin per Operation 6 by treatment with HF (5 ml) and anisole (0.25 ml) at 0°C for 1 hour. This process also removes the Bom protecting group from His, yielding final nonapeptides 3 and 6.

The peptides are purified with HPLC per Operation 7; their retention times are as follows. Analytical HPLC data

| Peptide No. | Gradient %B/min | Retention time on Column D |
|---|---|---|
| 3 | 25-65 | 15.71 |
| 6 | 25-65 | 17.37 |

Alternatively, the 5-membered heterocyclic ring may be formed in a solution by adding to 25 mg an intermediate peptide containing the structure of Leu-ψ-Cys-NH₂, 0.8 ml glacial acetic acid is mixed with 100 microliters 10% acetaldehyde at room temperature. This is mixed for 5 minutes; then 100 microliters ammonium acetate are added. The reaction mixture is subjected to purification to yield a desired peptide which contains the structure of Leu-ψ-MTac-NH₂.

### Example (4)

### Peptide #

18. D-Phe-Gln-Trp-Ala-Val-Gly-His-Leu-ψ-DMTac-NH₂
19. Ac-D-Phe-Gln-Trp-Ala-Val-Gly-His-Leu-ψ-DMTac
20. D-Cpa-Gln-Trp-Ala-Val-Gly-His-Leu-ψ-DMTac-NH₂
21. Tpi-Gln-Trp-Ala-Val-Gly-His-Leu-ψ-DMTac-NH₂ [SEQ ID NO: 9]
22. D-Tpi-Gln-Trp-Ala-Val-Gly-His-Leu-ψ-DMTac-NH₂

These polypeptides may suitably be synthesized from a common intermediate 1-4, Fmoc-Trp-Ala-Val-Gly-His(Bom)-Leu-ψ -Pen(But)-BHA (SEQ ID NO: 24) resin.

This intermediate is built step by step on benzhydrylamine (BHA) resin in accordance with the standard methods of solid phase synthesis as described in Examples (1 ) and (2).

Thus, 1.0 g BHA resin (0.55 mmole NH₂/g) is prepared according to Operation 2, being treated with 10 ml 10% TEA in CH₂Cl₂ (neutralization) twiceeach for three minutes and washed with 10 ml CH₂Cl₂ six times; then mixed with 1.6 mmole Fmoc-Pen(But) and 1.8 mmoles 1-hydroxybenzotriazole (HOBt) in DMF for three minutes. 20% 1 ,3-diisopropylcarbodiimide (DIC) with 1.6 mmoles in CH₂Cl₂ is added. The mixture is shaken at room temperature for 90 minutes. The resulting Fmoc-Pen(But)-BHA resin is washed with CH₂Cl₂, methanol two times each, and CH₂Cl₂ three times, and then subjected to a Kaiser test.

The removal of the Fmoc-group (deprotection) from Fmoc-Pen(But)-BHA resin is carried out per operation 4A.

The coupling of Fmoc-Leu-CHO is performed according to Operation 3. The A⁹(But)-BHA resin is washed with DMF 2 times. Then 1 .6 mmoles Fmoc-Leu- CHO in DMF containing 1 % AcOH is added, followed by 1.8 mmoles NaBH₃CN in DMF. The reaction mixture is shaken for 60 minutes, then washed with 50% methanol in H₂O 2 times, 100% MeOH 2 times, and CH₂Cl₂ 3 times, each for 1 minute.

After the removal of the Fmoc group from Fmoc-Leu-ψ-Pen(But)-BHA resin and neutralization, the coupling of Fmoc-His(Bom) is carried out as described as in Operation 4.

The coupling of Fmoc-Gly is performed as in Operation 4. 20% 1 ,3-diisopropyicarbodiimide (1.5 mmole) in CH₂Cl₂ was added to a DMF solution of 1.5 mmoles Fmoc-Gly and 1.65 mmoles HOBt at 0°C, stirred under cooling for 15 min and at room temperature for 15 min, the precipitate filtered off and added to resin, and shaken for 60 min. The subsequent amino acid residues Fmoc-Val, Fmoc-Ala and Fmoc-Trp are then sequentially introduced by coupling in the same manner to yield 1.9 g of intermediate 1-4, the protected peptide resin with structure Fmoc-Trp-Ala-Val-Gly-His(Bom)-Leu-ψ-Pen(But)-BHA (SEQ ID NO: 24) resin.

0.91 g of intermediate 1-4 is divided into five aliquots (about 200 mg each) which are used to synthesize protected polypeptide resins in accordance with the procedures described at Operation 4:
Sequential coupling of Fmoc-Gln and Boc-D-Phe to intermediate peptide 1-4 yields:
   Boc-D-Phe-Gln-Trp-Ala-Val-Gly-His(Bom)-Leu-ψ-Pen(But)-BHA resin ("4/18/A").
   Sequential coupling of Fmoc-Gln followed by Ac-D-Phe to intermediate peptide I- 4 yields: Ac-D-Phe-Gln-Trp-Ala-Val-Gly-His(Bom)-Leu-ψ-Pen(But)-BHA resin ("4/19/A").
   Sequential coupling of Fmoc-Gln and Boc-D-Cpa to intermediate peptide 1-4 yields:
Boc-D-Cpa-Gln-Trp-Ala-Val-Gly-His(Bom)-Leu-ψ-Pen(But)-BHA resin ("4/20/A").
   Sequential coupling of Fmoc-Gln followed by Boc-Tpi to intermediate peptide 1-4 yields: Boc-Tpi-Gln-Trp-Ala-Val-Gly-His(Bom)-Leu-ψ-Pen(But)-BHA (SEQ ID NO: 25) resin ("4/21 /A").
   Sequential coupling of Fmoc-Gln followed by Boc-Tpi to intermediate peptide 1-4 yields: Boc-D-Tpi-Gln-Trp-Ala-Val-Gly-His(Bom)-Leu-ψ-Pen(But)-BHA resin ("4/22/A").

The Boc-group is then removed from intermediate peptide 4/18/A with 50% TFA in CH₂Cl₂ containing 5% mercaptoethanol and 5% anisole, resulting in intermediate peptide 4/18/B: D-Phe-Gln-Trp-Ala-Val-Gly-His(Bom)-Leu-ψ-Pen-BHA resin.

At this point, the Pen side chain of intermediate peptide 4/18/B is cyclized by oxidation to form the 5-membered heterocyclic ring shown in Formula IIC per Operation 5. A 10 ml mixture of AcOH, 3.7% HCHO and DMF (1 :1 :8) is added to form a reaction mixture which is shaken at room temperature for 3 minutes, washed with water, DMF and CH₂Cl₂ 3 times each to yield intermediate peptide 4/18/C: D-Phe-Gln-Trp-Ala-Val-Gly-His(Bom)-Leu-ψ-DMTac-BHA resin.

Intermediate peptide 4/18/C is then washed with 5 ml CH₂Cl₂, methanol and CH₂Cl₂ three times each and treated with freshly distilled HF (5ml) and anisole (0.25 ml) at 0°C for 1 hr. The solvent is evaporated in vacuo, washed with ether or ethylacetate then extracted with 70-80% acetic acid and lyophilized to yield crude nonapeptide resin. The reaction mixture is purified to yield bombesin antagonist peptide number 18.

The same steps for removing the protecting groups, cyclizing the deprotected A⁹ and displacing the intermediate peptide from the BHA resin are performed on intermediate peptides 4/19/A, 4/20/A, 4/21 /A, and 4/22/A to yield peptide numbers 19, 20, 21 and 22.
The purification is carried by HPLC with solvent system consisting of (A) % TFA and (B) 1 % TFA in 70% acetonitrile in accordance with Operation 7. Purified peptides are proved to be over 97% pure in analytical HPLC. Retention time of these peptides follow.

| Peptide No. | Gradient %B/min | Retention time on Column D |
|---|---|---|
| 18 | 25-65 | 17.23 |
| 19 | 25-65 | 19.13 |
| 20 | 25-65 | 20.34 |
| 21 | 25-65 | 19.13 |
| 22 | 25-65 | 20.34 |

### Example (3)

Alternatively, the A⁹ residue may be cyclized in solution by adding 25 mg free peptide containing the structure -Leu-ψ-Pen-NH₂ to 25 mg HOBt in 0.8 ml glacial acetic acid mixed with 100 microliters 10% formaldehyde and stirring at 0°C for 30 minutes to yield the peptide having the 5-membered ring structure Leu-ψ-DMTac-NH₂.

### Example (5)

### Peptide #

23. Pal-Gln-Trp-Ala-Val-Gly-His-Leu- ψ-Cys-NH₂ [SEQ ID NO: 10]
24. D-Pal-Gln-Trp-Ala-Val-Gly-His-Leu-ψ-Cys-NH₂
25. Tpi-Gln-Trp-Ala-Val-Gly-His-Leu-ψ-Cys-NH₂ [SEQ ID NO: 11]
26. D-Tpi-Gln-Trp-Ala-Val-Gly-His-Leu-ψ-Cys-NH₂
27. Hca-Gln-Trp-Ala-Val-Gly-His-Leu-ψ-Cys-NH₂ [SEQ ID NO: 12]

These peptides may be synthesized from a common intermediate 1-5, Fmoc-Gln-Trp-Ala-Val-Gly-His(Bom)-Leu-ψ-Cys(But)-BHA (SEQ ID NO: 23) resin.

Fmoc-Leu-ψ-Cys(But)-BHA resin is obtained as follows: 1.0 g BHA resin (0.55m mole NH₂/g) is coupled with Fmoc-Cys(But) and Fmoc-Leu-CHO successively by the method indicated in Operations 2 and 3.

Sequential coupling of Fmoc-His(Bom), Fmoc-Gly, Fmoc-Val, Fmoc-Ala and Fmoc-Trp and Fmoc-Gln results in Fmoc-Gln-Trp-Ala-Val-Gly-His(Bom)-Leu-ψ-Cys(But)-BHA (SEQ ID NO: 23) resin, intermediate peptide 1-5.

A 150 mg. aliquot of this intermediate 1-5 common to all peptides of this Example, is subjected to one further coupling with the procedures described in Operation 4 to yield the final peptide resins.
Sequential coupling of Boc-Pal to intermediate peptide 1-5 yields: Boc-Pal-Gln- Trp-Ala-Val-Gly-His(Bom)-Leu-ψ-Cys(But)-BHA (SEQ ID NO: 17) resin ("5/08/A").
Sequential coupling of Boc-D-Pal to intermediate peptide 1-5 yields: Boc-D-Pal- Gln-Trp-Ala-Val-Gly-His(Bom)-Leu-ψ-Cys(But)-BHA resin ("5/09/A").
Sequential coupling of Boc-Tpi to intermediate peptide 1-5 yields: Boc-Tpi-Gln- Trp-Ala-Val-Gly-His(Bom)-Leu-ψ-Cys(But)-BHA (SEQ ID NO: 18) resin ("5/12/A").
Sequential coupling of Boc-D-Tpi to intermediate peptide 1-5 yields: Boc-D-Tpi- Gln-Trp-Ala-Val-Gly-His(Bom)-Leu-ψ-Cys(But)-BHA resin ("5/13/A").
Sequential coupling of Hca to intermediate peptide 1-5 yields: Hca-Gln-Trp-Ala- Val-Gly-His(Bom)-Leu-ψ-Cys(But)-BHA (SEQ ID NO: 19) resin ("5/14/A").

The N-terminal Boc group is removed from intermediate peptide 5/01 /A by treatment with 50% TFA in CH₂Cl₂ containing 5% mercaptoethanol and 5% anisole twice, first for 5 minutes and then for 25 minutes. This treatment removes the protecting group from Cys. The peptide is then washed with CH₂Cl₂, MeOH and DMF, per Operation 4, resulting in intermediate peotide 5/08/B: Pal-Gln-Trp-Ala-Val-Gly-His(Bom)-Leu-ψ-Cys-BHA (SEQ ID NO: 26) resin.

Intermediate peptide 5/08/B is then treated with freshly distilled HF (5 ml) and anisole (0.25 ml) at 0°C for 1 hour, also removing the Bom protecting group from His. The solvent is evaporated in vacuo and washed with ethylacetate, extracted with 70-80% acetic acid and lyophilized, yielding the following peptide: Pal-Gln-Trp-Ala-Val-Gly-His-Leu-ψ-Cys-NH₂ (SEQ ID NO: 10).

The protecting group and BHA resin are removed from intermediate peptides 5/08/A, 5/09/A, 5/11 /A, 5/12/A 5/13/A and 5/14/A to yield the peptides enumerated at the beginning of this Example. These are subjected to purity test in accordance with Operation 7.

### Peptide No.23-27

Alternatively, these peptides may suitably be built step-by-step on BHA resin with Boc-protected amino acids and using Bz to protect the -SH group of Cys⁹ in accordance with the standard methods of solid phase methods, resulting in intermediate peptides as follows:
Boc-Pal-Gln-Trp-Ala-Val-Gly-His(Bom)-Leu-ψ-Cys(Bz)-BHA (SEQ ID NO: 27) resin ("5/08/A"). Boc-D-Pal-Gln-Trp-Ala-Val-Gly-His(Bom)-Leu-ψ-Cys(Bz)-BHA resin ("5/09/A").
Boc-Tpi-Gln-Trp-Ala-Val-Gly-His(Bom)-Leu-ψ-Cys(Bz)-BHA (SEQ ID NO: 28) resin ("5/12/A").
Boc-D-Tpi-Gln-Trp-Ala-Val-Gly-His(Bom)-Leu-ψ-Cys(Bz)-BHA resin ("5/13/A").
Hca-Gln-Trp-Ala-Val-Gly-His(Bom)-Leu-ψ-Cys(Bz)-BHA (SEQ ID NO: 29) resin ("5/14/A")

The Boc group is removed by treatment with 50% TFA in CH₂Cl₂ containing 5% mercaptoethanol and 5% anisole, and then treated with HF and anisole to cleave the peptide from the resin, also removing the protecting group Bom from His and Bz from Cys to yield the peptides enumerated at the beginning of this example.

### Example (6): Assay Procedures (A) Receptor Binding Assay

Human SK-Hep-1 cancers were transplanted sc. into both flank areas of female nude mice. The treatment started 47 days after transplantation as follows: Group 1: Control; Group 2: Peptide 14, 20 µg/day;. There were 8 animals in each group. The substances were dissolved in 0.1% dimethyl sulfoxide (DMSO) and diluted with 10% propylene glycol in water. Amounts of 0.1 ml of the solutions were injected once daily sc. The controls received vehicle only. Tumors and body weights were measured weekly and tumor volume was calculated. The experiment was terminated on day 135.

SK-Hep-1 tumor grew very slowly, therefore the experiment lasted for almost 6 months. The mice tolerated well the long treatment, body and organ weights were not changed by any therapy.

After 35 days, treatment with Peptide 14 resulted in a 94% decrease in tumor volume with a total regression of some tumors. The mean tumor volume in Group 2 remained below the initial value from day 50 until the end of the experiment.

Spontaneous regression occasionally occurs in untreated SK-Hep-1 cancers. The numbers of totally regressed tumors in the various groups are shown in the Table. Since bombesin antagonist Peptide 14 has no toxic effects at therapeutic doses, it can be used for treatment of human patients with liver cancer.

**Table 1. Effect of treatment with bombesin antagonist Peptide 14.**

| Group | Tumor volume (mm³) on day 135 | Tumor weights (mg) | Total number of tumors in groups | Tumors with total regression |
|---|---|---|---|---|
| 1. Control | 730 ± 434 | 1388 ± 843 | 10 | 1 |
| 2. Peptide 14 | 47 ± 39* | 160 ± 142 | 10 | 4 |

| | | | | |
|---|---|---|---|---|
| Volume and weight values are mean ± SE *P=0.009 vs. control | | | | |

### Example 7

Hep-G2 cancers were transplanted sc. into both flank areas of 60 female nude mice. The treatment started 29 days after transplantation as follows: Group 1: Control; Group 2: Peptide 14, 10 µg/day. There were 10 animals in the groups. The compound was given once daily sc. for 69 days. The experiment was terminated on day 127.

### Results

The tumors grew very fast in some mice and slower in others, thus on day 51, 5 mice with the largest tumors in each group were sacrificed. Tumor volume changes and tumor growth rate are shown in the Figures and volume and doubling time data in the Table. Tumor weights cannot be compared because the animals were sacrificed at different time points. Peptide 14at 10 µg daily doses significantly inhibited growth of Hep-G2 cancers. Body and organ weights were not changed by the treatments.

### Conclusion

Bombesin antagonist Peptide 14 at 10 µg/day doses significantly inhibited growth of Hep-G2 hepatocellular cancers in nude mice.

**Table 2. Effect of treatment with bombesin antagonist Peptide 14 on growth of Hep-G2 human hepatocellular cancers in nude mice**

| Group | Number of tumors on day 1/day 52 | Tumor volume on day 51 (mm³) | Tumor volume on day 106 (mm³) | Tumor doubling time (days) |
|---|---|---|---|---|
| 1. Control | 11/5 | 2730±1046 | 3608±682 | 13.37±1.45 |
| 2. Peptide 14, 10 µg/day | 10/6 | 886±334 | 1019±387* | 17.79±1.36 * |

| | | | | |
|---|---|---|---|---|
| *P<0.05 vs Control | | | | |

## Claims

1. The use of a bombesin antagonist peptide having the formula:
X-A¹-A²-Trp-Ala-Val-Gly-His-Leu-ψ-A⁹ -Q (SEQ ID NO: 32)
wherein X is hydrogen, Hca, Hna, Hpp, Mpp or Naa,
A¹ is a D-, L- or DL-amino acid residue selected from the group consisting of Phe, p-HI-Phe, pGlu, Nal, Pal, Tpi, unsubstituted Trp or Trp substituted in the benzene ring by one or more members selected from the group consisting of F, Cl, Br, NH₂ or C₁₋₃ alkyl; or is a peptide bond linking the acyl moiety of X to the alpha amino moiety of A²,
A² is Gin, Glu [-], Glu (Y), or His, wherein
[-] is a single bond, linking the gamma carboxyl moiety or the 3-propionyl moiety of A² with the alpha amino moiety of A¹,
Y is
a) -OR⁵ wherein R⁵ is hydrogen, C₁₋₃ alkyl or phenyl, or
b) 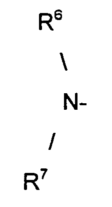 wherein R⁶ is hydrogen or C₁₋₃ alkyl; R⁷ is hydrogen, C₁₋₃ alkyl or -NHCONH₂,
Leu-ψ- is a reduced form of Leu, wherein the C = O moiety of Leu is instead -CH₂-such that the bond of this -CH₂- moiety with the alpha amino moiety of the adjacent A⁹ residue is a pseudopeptide bond,
A⁹ is Tac, MTac, or DMTac,
Q is NH₂ or -OQ¹ where Q¹ is hydrogen, C₁₋₁₀ alkyl, phenyl or phenyl-C₁₋₁₀ -alkyl; and the pharmaceutically acceptable acids or salts thereof for the manufacture of a
medicament for treating human hepatomas in a patient in need of same.

2. The use of claim 1 wherein the peptide has the formula wherein
A¹ is D-Cpa, D-Nal, L- or D-Pal, D-Phe, L- or D-Tpi, or D-Trp,
A² is Gln or His, and
Q is NH₂.

3. The use of claim 2 wherein the peptide has the formula wherein
A⁹ is Tac.

4. The use of claim 2 wherein the peptide has the formula wherein
A⁹ is DMTac.

5. The use of claim 3 wherein the peptide has the formula wherein
X is H or Ac,
A¹is D-Phe, L- or D-Tpi, and
A² is Gln.

6. The use of claim 1 wherein the peptide has the formula wherein
A² is Gln,
A⁹ is Tac, and
Q is NH₂.

7. The use of claim 1 wherein the peptide has the formula wherein
A¹ is D- or L- residue selected from the group consisting of Phe, p-HI-Phe and Trp.

8. The use of claim 3 wherein the peptide has the formula

D-Phe-Gln-Trp-Ala-Val-Gly-His-Leu-ψ-Tac- NH₂ , or D-Tpi-Gln-Trp-Ala-Val-Gly-His-
Leu-ψ-Tac- NH₂.

9. The use of claim 1 wherein the peptide has the formula wherein
X is Hca, Hna, Hpp, Mpp or Naa,
A¹ is a peptide bond linking the acyl moiety of X to the alpha amino moiety of A²,
A² is Gln and
A⁹ is Tac.

10. The use of claim 9 wherein the peptide has the formula
Hca-Gln-Trp-Ala-Val-Gly-His-Leu-ψ-Tac-NH₂ [SEQ ID NO: 7].

11. The use of claim 1 wherein the peptide is selected from the group having the formulae
D-Phe-Gln-Trp-Ala-Val-Gly-His-Leu-ψ-Tac-NH₂ ,
D-Phe-Gln-Trp-Ala-Val-Gly-His-Leu-ψ-MTac-NH₂ ,
Ac-D-Phe-Gln-Trp-Ala-Val-Gly-His-Leu-ψ-Tac-NH₂,
D-Trp-Gln-Trp-Ala-Val-Gly-His-Leu-ψ-Tac-NH₂,
Ac-D-Trp-Gln-Trp-Ala-Val-Gly-His-Leu-ψ-Tac-NH₂,
D-Tpi-Gln-Trp-Ala-Val-Gly-His-Leu-ψ-Tac-NH₂,
D-Phe-His-Trp-Ala-Val-Gly-His-Leu-ψ-Tac-NH₂,
D-Phe-Gln-Trp-Ala-Val-Gly-His-Leu-ψ-DMTac-NH₂.

12. A use of a bombesin antagonist peptide having the formula:
X-A¹-A²-Trp-Ala-Val-Gly-His-Leu-ψ-A⁹ -Q (SEQ ID NO: 33)
wherein X is hydrogen, Hca, Hna, Hpp, Mpp or Naa,
A¹ is a D-, L- or DL-amino acid residue selected from the group consisting of Phe, p-HI-Phe, pGlu, Nal, Pal, Tpi, unsubstituted Trp or Trp substituted in the benzene ring by one or more members selected from the group consisting of F, Cl, Br, NH₂ or C₁₋₃ alkyl; or is a peptide bond linking the acyl moiety of X to the alpha amino moiety of A²;
A² is Gin, Glu [-], Glu (Y), or His, wherein
[-] is a single bond, linking the gamma carboxyl moiety or the 3-propionyl moiety of A² with the alpha amino moiety of A¹,
Y is
a) -OR⁵ wherein R⁵ is hydrogen, C₁₋₃ alkyl or phenyl; or
b) 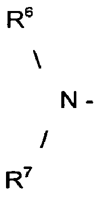
wherein R⁶ is hydrogen or C₁₋₃ alkyl; R⁷ is hydrogen, C₁₋₃ alkyl or -NHCONH₂, Leu-ψ- is a reduced form of Leu, wherein the C = O moiety of Leu is instead -CH₂-such that the bond of this -CH₂- moiety with the alpha amino moiety of the adjacent A⁹ residue is a pseudopeptide bond,
A⁹ is Cys or Pen,
Q is NH₂ or -OQ¹ where Q¹ is hydrogen, C₁₋₁₀ alkyl, phenyl or phenyl-C₁₋₁₀ -alkyl, and the pharmaceutically acceptable acids or salts thereof for the manufacture of a medicament for treating human hepatomas in a patient in need of same
